# EUROPÄISCHE PATENTSCHRIFT

(11) **EP 4 514 306 B1**
(45) Veröffentlichungstag und Bekanntmachung des Hinweises auf die Patenterteilung: **09.12.2026**
(21) Anmeldenummer: 23715797.9
(22) Anmeldetag: 27.03.2023
(51) Int. Cl.: A61K 8/02, A61K 8/19, A61K 8/20, A61K 8/24, A61K 8/44, A61K 8/46, A61K 8/73, A61Q 5/08

(54) **OXIDATIVES HAARAUFHELLUNGSMITTEL MIT VERBESSERTEN ANWENDUNGSEIGENSCHAFTEN FÜR DIE BLONDIERUNG DES HAARANSATZES**
OXIDATIVE HAIR-LIGHTENING AGENTS HAVING IMPROVED APPLICATION PROPERTIES FOR BLEACHING HAIR ROOTS
AGENT D'ÉCLAIRCISSEMENT CAPILLAIRE PAR OXYDATION PRÉSENTANT DES PROPRIÉTÉS D'APPLICATION AMÉLIORÉES POUR DÉCOLORER LA RACINE DES CHEVEUX

(30) Priorität: 27.04.2022 DE 102022204090
(43) Veröffentlichungstag der Anmeldung: 05.03.2025
(73) Patentinhaber: Henkel AG & Co. KGaA, 40589 Düsseldorf (DE)
(72) Erfinder: ANDERHEGGEN, Bernd, 41189 Mönchengladbach (DE); REICHERT, Anja, 40629 Düsseldorf (DE)
(86) Internationale Anmeldenummer: PCT/EP2023/057797
(87) Internationale Veröffentlichungsnummer: WO 2023/208489

(56) Entgegenhaltungen:
- EP-A2- 3 572 126
- DE-A1- 102018 123 507
- FR-A1- 2 842 099
- FR-A1- 2 925 846
- FR-A1- 2 994 647
- JP-A- H0 967 235
- US-A1- 2017 290 749

## Beschreibung

Die vorliegende Erfindung betrifft oxidative Haaraufhellungsmittel, insbesondere Blondierpulver und Blondierpasten, die speziell zur Anwendung auf dem Haaransatz, das heißt, auf dem Wurzel-nahen Teil der Haarfasern, geeignet sind.

Weiterhin betrifft die vorliegende Erfindung die Verwendung der Mittel zur Blondierung bzw. oxidativen Aufhellung oder zum aufhellenden Färben von menschlichen Haaren, insbesondere am Haaransatz, sowie eine Mehrkomponentenverpackungseinheit (Kit-of-Parts) zur Aufhellung oder zum aufhellenden Färben keratinischer Fasern, die ein oxidatives Haaraufhellungsmittel und, davon getrennt, eine Oxidationsmittelzubereitung umfasst.

Weiterhin wird ein Verfahren zur oxidativen Aufhellung oder zum aufhellenden Färben von keratinischen Fasern unter Verwendung des genannten oxidativen Haaraufhellungsmittels und des Kits, das dieses oxidative Haaraufhellungsmittel umfasst, beschrieben.

Bei dem erfindungsgemäßen oxidativen Haaraufhellungsmittel handelt es sich eine im Wesentlichen wasserfreie Oxidationsmittelzubereitung, enthaltend mindestens ein Persalz. Das im Wesentlichen wasserfreie oxidative Haaraufhellungsmittel liegt in nicht-komprimierter Pulverform oder als fließfähige Paste vor.

Das Aufhellen der eigenen Haarfarbe ist seit jeher der Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Die in diesen Produkten enthaltenen Oxidationsmittel sind in der Lage, durch die oxidative Zerstörung des haareigenen Farbstoffs Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein. Für das Erzielen eines stärkeren Blondiereffekts wird üblicherweise eine Mischung aus Wasserstoffperoxid und mindestens einer Verbindung, ausgewählt aus Persalzen, insbesondere Peroxodisulfatsalzen und/oder Peroxomonosulfatsalzen, eingesetzt. Zur Verstärkung der Blondierwirkung enthalten die Mittel höhere Einsatzkonzentrationen an Wasserstoffperoxid und Persalzen, insbesondere Persulfaten. Dunkles, dunkelbraunes oder schwarzes Haar lässt sich so in einem Schritt um 4 bis 6 Nuancen aufhellen. Das Wasserstoffperoxid und das Persalz-haltige Haaraufhellungsmittel werden bis zur Anwendung getrennt voneinander aufbewahrt, um die Persalze nicht vorzeitig zu deaktivieren. Die Wasserstoffperoxid-Komponente, die eine wässrige Lösung von Wasserstoffperoxid umfasst, weist zur Stabilisierung des Wasserstoffperoxids einen sauren pH-Wert, insbesondere einen pH-Wert von 1,5 bis 5,5, insbesondere von 3 bis 5, auf, jeweils bei 20°C gemessen.

Für die Melanin abbauende Wirkung des Wasserstoffperoxids und die Blondierwirkung auf der keratinischen Faser ist es jedoch vorteilhaft, wenn die Anwendungsmischung aus Wasserstoffperoxid-Lösung und Persalz-haltigem Haaraufhellungsmittel einen alkalischen pH-Wert besitzt, der im Bereich von 8,0 bis 11,5, bevorzugt im Bereich von 8,5 bis 11,0, besonders bevorzugt 9,0 - 10,5, außerordentlich bevorzugt 9,5 - 10,0 liegt, jeweils gemessen bei 20°C.

Zur Einstellung eines alkalischen pH-Werts der aufhellenden Anwendungsmischung können erfindungsgemäß bevorzugte oxidative Haaraufhellungsmittel neben dem mindestens einen Persalz mindestens ein pulverförmiges Alkalisierungsmittel in einer solchen Gesamtmenge enthalten, dass die Anwendungsmischung den gewünschten alkalischen pH-Wert aufweist. Alternativ kann man der Anwendungsmischung aus erfindungsgemäßem oxidativen Haaraufhellungsmittel und Wasserstoffperoxid-Lösung eine dritte Zusammensetzung zusetzen, die ein oder mehrere Alkalisierungsmittel in einer solchen Menge enthält, dass die Anwendungsmischung einen alkalischen pH-Wert besitzt, der im Bereich von 8,0 bis 11,5, bevorzugt im Bereich von 8,5 bis 11,0, besonders bevorzugt 9,0 - 10,5, außerordentlich bevorzugt 9,5 - 10,3 liegt, jeweils gemessen bei 20°C.

Setzt man dem oxidativen Haaraufhellungsmittel Oxidationsfarbstoffvorprodukte und/oder direktziehende Farbstoffe zu, so kann das Haar gleichzeitig gefärbt werden. Entsprechende Haarfärbemittel werden insbesondere für Verbraucher mit sehr dunklem Melanin-reichen Haar angeboten.

Das durchschnittliche Haarwachstum liegt bei ca. 0,3 bis 0,5 mm pro Tag und etwa 1 bis 1,5 cm pro Monat. Das heißt, dass bereits 2 bis 4 Wochen nach einer Aufhellbehandlung des Haupthaars die einzelnen Haarfasern so weit nachgewachsen sind, dass man die natürliche Haarfarbe und damit den Unterschied zwischen der aufgehellten und der natürlichen Haarfarbe deutlich erkennen kann. Dies halten viele Verbraucher für unansehnlich. Eine Wiederholung der Blondierung am gesamten Haupthaar würde zwar den dunkel oder grau nachgewachsenen Haaransatz blondieren, aber die noch blondierten Haarlängen unnötigerweise schädigen.

Es wäre daher wünschenswert, ein Haaraufhellungsmittel bereitzustellen, das speziell auf die Anwendung am Haaransatz, also in der Nähe der Kopfhaut, hin optimiert ist. Dabei muss das Blondiermittel einerseits stark genug wirken, um das ungeschädigte, stabile und damit besonders schwer aufzuhellende Ansatzhaar wirksam aufzuhellen. Andererseits muss das Hautirritationspotenzial des anwendungsbereiten Blondiermittels möglichst gering gehalten werden, da bei der Applikation des Mittels auf das Ansatzhaar unweigerlich auch zumindest kleine Mengen des Mittels mit der Kopfhaut in Kontakt kommen können. In diesem Zusammenhang sollte angestrebt werden, die Blondierung mit einer geringen bis mittleren Wasserstoffperoxidkonzentration durchzuführen, die etwa im Bereich von 2,5 bis 4,5 Gew.-%, bevorzugt 3 - 4 Gew.-% Wasserstoffperoxid *on head* liegen sollte.

Damit bei der Applikation des Mittels nur möglichst geringe Mengen des anwendungsbereiten Blondiermittels auf die Kopfhaut fließen können, muss das anwendungsbereite Mittel eine cremige, kompakte Konsistenz aufweisen, und es muss sich möglichst schnell eine Haut oder Kruste bilden, die das Herunterfließen auf die Kopfhaut unterbindet.

Da es beim Vermischen des wasserfreien Blondiermittels (nicht-komprimiertes Pulver oder Paste) mit der wässrigen Wasserstoffperoxidlösung zu chemischen Reaktionen kommt, bei denen gasförmiger Sauerstoff freigesetzt wird, quillt das anwendungsbereite Blondiermittel während der Einwirkzeit auf dem Haar mehr und mehr auf. Es verändert sich von anfänglich einer Creme hin zu einer schaumigen Quellung. Durch die grobporige Schaumtextur haftet das anwendungsbereite Blondiermittel nicht genügend an der Haarfaser. Dies bedeutet, dass weniger anwendungsbereites Blondiermittel und damit auch weniger Blondierwirkstoff an die Haarfaser gelangen. Um die Schaumbildung zu minimieren, empfiehlt es sich, den Tensidgehalt im Blondiermittel möglichst gering zu halten oder aber das Mittel frei von Tensiden und Emulgatoren zu formulieren. Das kann zu einem Zielkonflikt führen, da die anwendungsbereiten Blondiermittel häufig mit einem gewissen Gehalt an Öl und/oder Fettkomponenten formuliert werden, um die Haarschädigung während der harschen oxidativen Haarbehandlung zu minimieren. Um Öl und Fettkomponenten mit der wässrigen Wasserstoffperoxidlösung mischbar zu machen, kann allerdings manchmal ein Gehalt an Emulgatoren unumgänglich sein. Bestimmte Siliconverbindungen können die Schaumbildung unterdrücken, aber die vorliegenden Blondiermittel sollten aus Gründen der ökologischen Nachhaltigkeit ohne den Zusatz von Siliconverbindungen formuliert werden.

Neben einem Gehalt an Tensiden oder Emulgatoren sind vor allem die im wasserfreien Blondierpulver oder in der wasserfreien Blondierpaste verwendeten Verdickungsmittel für die Schaumbildung verantwortlich. Diese Verdickungsmittel, bei denen es sich im Wesentlichen um hydrophile polymere Verdickungsmittel handelt, führen beim Vermischen des wasserfreien Blondiermittels (nicht-komprimiertes Pulver oder Paste) mit der wässrigen Wasserstoffperoxidlösung dazu, dass das anwendungsbereite Blondiermittel eine cremeähnliche Konsistenz annimmt, die sich zunächst gut auf das Haar auftragen lässt, sich dann aber während der Einwirkzeit auf dem Haar möglichst verfestigt, damit sie nicht vom Haar heruntertropft oder auf die Kopfhaut fließt.

Eine weitere Vorgabe für die vorliegenden Blondiermittel war der Verzicht auf Polymere, die als Kettenbildendes Monomer Acrylsäure, Acrylsäureamide, Acrylsäureester, Methacrylsäure, Methacrylsäureamide, Methacrylsäureester, Vinylpyrrolidone, Vinylacetat, Vinylalkohol und/oder Styrole umfassen. Daher sollten die hydrophilen polymeren Verdickungsmittel auf natürlichen Rohstoffen basieren.

Weiterhin sollte sich beim Vermischen des wasserfreien Blondiermittels mit der wässrigen Wasserstoffperoxidlösung möglichst wenig Reaktionswärme entwickeln, um die Kopfhaut nicht thermisch zu reizen. In Vorversuchen konnte gezeigt werden, dass die Wärmeentwicklung durch die Wahl des Komplexbildners beeinflusst werden kann. Aber auch hier spielt die Wahl des Verdickungsmittels eine gewisse Rolle.

Der Einsatz von Komplexbildnern in höherer Konzentration zur Reduzierung von Haarschäden bei der oxidativen Haarbehandlung war bereits bekannt aus WO2002089754A1, WO2002074272A1 und WO2002074273A1. EP1714634A1 offenbart ein Haarbehandlungs-Kit zum Färben von menschlichen Haaren, umfassend ein erstes Kompartiment mit einem Komplexbildner und ein zweites Kompartiment mit einem Färbemittel. Durch den Komplexbildner sollten unerwünschte Reaktionen an und mit Haaren, die zu einer unerwünschten Erwärmung des Mittels während der Herstellung der Anwendungsmischung und deren Einwirkzeit auf dem Haar führen, unterbunden werden.

In vielen auf dem Markt befindlichen Blondierprodukten sind als Komplexbildner HEDP (1-Hydroxyethan-1,1-diphosphonsäure, Etidronsäure) oder deren Salze oder aber EDTA (Ethylendiamintetraessigsäure) oder deren Salze enthalten. HEDP wie auch EDTA stabilisieren Wasserstoffperoxid auf effektive Weise und komplexieren vorhandene Metallionen so effizient, dass eine bei der Anwendung stattfindende unerwünschte Temperaturerhöhung quasi vollständig vermieden wird. Ein großer Nachteil von HEDP und EDTA liegt jedoch in ihrer schlechten biologischen Abbaubarkeit. Gerade in jüngster Zeit achten Verbrauchende zunehmend auf das ökologische Profil der von ihnen verwendeten kosmetischen Produkte. So bevorzugen Verbrauchende vor allem möglichst nachhaltige Kosmetika mit biologisch abbaubaren Inhaltsstoffen.

Auch der Einsatz von Substanzen natürlicher Herkunft als Komplexbildner ist im Stand der Technik bereits bekannt. So offenbart FR2852834A1 Mittel zum Blondieren, Färben bzw. Verformen von Haaren, die die Kombination aus mindestens einem Oxidationsmittel und mindestens einer Polyhydroxycarbonsäure enthalten. Auch wenn die Polyhydroxycarbonsäuren der FR2852834A1 biologisch gut abbaubar sind, so sind die anwendungstechnischen Eigenschaften der in dieser Schrift beschriebenen Mittel immer noch verbesserungswürdig, gerade in Bezug auf die Wärmeentwicklung, die in Anwesenheit von Gluconsäure und ihren Salzen besonders ungünstig verläuft.

Der Einsatz von mikrokristalliner Cellulose in Blondiermitteln war bereits bekannt aus WO2012079952A2 sowie aus FR2994647A1, FR2994649A1 und weiteren Anmeldungen dieser Serie, allerdings offenbaren diese Schriften das Blondiermittel in komprimierter Form als Tablette, wobei die mikrokristalline Cellulose in hohen Konzentrationen von mehr als 10 Gew.-% enthalten ist und als Sprengmittel dient, um den Zerfall der Tablette in Wasser zu beschleunigen.

EP3572126A2 offenbart ein wasser- und siliconfreies Blondierpulver mit Ammoniumpersulfat, Kaliumpersulfat, Xanthan und dem kationisierten Guar-Derivat Guarhydroxypropyltrimoniumchlorid. JPH0967235A offenbart wasser- und siliconfreie Blondierpulver, die Persulfate und Xanthan enthalten. FR2842099A1 offenbart wasser- und siliconfreie Blondierpulver, die Persulfate, Xanthan und Guar enthalten. US2017290749A1 offenbart ein wasser- und siliconfreies Blondierpulver mit Ammoniumpersulfat, Kaliumpersulfat, Xanthan, Cyamopsis Tetragonoloba Gum und dem kationisierten Guar-Derivat Guarhydroxypropyltrimoniumchlorid. FR2925846A1 offenbart ein wasser- und siliconfreies Blondierpulver mit Kaliumpersulfat, Xanthan und Guar.

Es war die Aufgabe der vorliegenden Erfindung, oxidative Haaraufhellungsmittel, insbesondere Blondiermittel bzw. Mittel zur oxidativen Farbveränderung, bereitzustellen, die für die Anwendung am Haaransatz, also in der Nähe der Kopfhaut, optimal geeignet sind. Dabei muss das Blondiermittel einerseits stark genug wirken, um das ungeschädigte, stabile und damit besonders schwer aufzuhellende Ansatzhaar wirksam aufzuhellen. Andererseits muss das Hautirritationspotenzial des anwendungsbereiten Blondiermittels möglichst gering gehalten werden, da bei der Applikation des Mittels auf das Ansatzhaar unweigerlich auch zumindest kleine Mengen des Mittels mit der Kopfhaut in Kontakt kommen können. Dafür sollte das anwendungsbereite Mittel nach der Applikation auf das Haar eine cremig-kompakte Konsistenz entwickeln und eine Kruste ausbilden. Weiterhin sollte die Wärmeentwicklung bei der Herstellung des anwendungsbereiten Blondiermittels minimiert werden. Die dabei eingesetzten Inhaltsstoffe sollten ökologisch möglichst nachhaltig sein.

Überraschend wurden diese Aufgaben durch den Gegenstand der Ansprüche gelöst.

Ein erster Gegenstand der vorliegenden Erfindung ist ein oxidatives Haaraufhellungsmittel zum Aufhellen oder zum aufhellenden Färben der Haare, das in nicht-komprimierter Pulverform oder als fließfähige Paste vorliegt, enthaltend
a) mindestens ein Oxidationsmittel, ausgewählt aus den anorganischen Salzen einer Peroxoschwefelsäure, sowie Mischungen dieser Salze,
b) Xanthan,
c) ein Polysaccharid, ausgewählt aus mikrokristalliner Cellulose und Mischungen von mikrokristalliner Cellulose und Guar Gum,
d) und 0 bis 10 Gew.-%, bevorzugt 0,1 bis 8 Gew.-%, besonders bevorzugt 0,5 bis 5 Gew.-% Wasser, jeweils bezogen auf das Gewicht des oxidativen Haaraufhellungsmittels,
e) wobei keine Siliconverbindungen enthalten sind.

Das mit 0 bis 10 Gew.-% Wasser im Wesentlichen wasserfreie oxidative Haaraufhellungsmittel ist nichtkomprimiert pulverförmig oder pastenförmig.

Unter dem Begriff "Pulver" oder "pulverförmig" ist erfindungsgemäß eine bei 20 °C und 1013 mbar (101.3 kPa) feste, rieselfähige Darreichungsform aus einzelnen Partikeln zu verstehen, bei der die einzelnen Partikel Partikelgrößen im Bereich von 0,1 µm bis maximal 1,6 mm aufweisen. Die Bestimmung der Partikelgrößenverteilung kann bevorzugt mittels Laserbeugungsmessung gemäß ISO 13320-1 (2009) erfolgen. Gegebenenfalls können die Partikel durch physikalische Behandlung, wie Sieben, Pressen, Granulieren oder Pelletieren, oder durch den Zusatz bestimmter Hilfsstoffe in ihrer Korngröße den Anforderungen an das Blondierpulver angepasst werden, um beispielsweise eine bessere Mischbarkeit der einzelnen Pulverbestandteile oder die Mischbarkeit des Blondierpulvers mit einer Wasserstoffperoxid-Zubereitung zu ermöglichen.

Erfindungsgemäß bevorzugte Blondierpulver weisen eine Schüttdichte im Bereich von 400 bis 1000 g/l (Gramm/Liter), bevorzugt 450 bis 900 g/l, besonders bevorzugt 550 bis 820 g/l, auf. Die Bestimmung der Schüttdichte erfolgt bevorzugt nach EN ISO 60 (Version 01/2000) oder DIN ISO 697 (Version 01/1984). Sofern nicht anders angegeben, beziehen sich alle Temperaturangaben auf einen Druck von 1013 mbar (101.3 kPa).

Unter den Begriffen "Paste" oder "pastenförmig" ist erfindungsgemäß eine Darreichungsform zu verstehen, die bei 20 °C und 1013 mbar (101.3 kPa) eine Viskosität im Bereich von 200.000 bis 1.600.000 mPas, bevorzugt 250.000 bis 1.400.000 mPas, besonders bevorzugt 300.000 bis 1.000.000 mPas, außerordentlich bevorzugt 400.000 bis 750.000 mPas, aufweist.

Die Bestimmung der Pastenviskosität erfolgt bevorzugt mittels Brookfield; Gerät RVDV II+; Spindel Nr. 6, 4 Umdrehungen pro Minute, bei 20°C.

Das erfindungsgemäße oxidative Haaraufhellungsmittel enthält als ersten wesentlichen Bestandteil mindestens ein Oxidationsmittel, das aus den anorganischen Salzen einer Peroxoschwefelsäure sowie Mischungen hiervon ausgewählt ist.

Unter Peroxoschwefelsäuren werden Peroxodischwefelsäure und Peroxomonoschwefelsäure (Caro'sche Säure) verstanden.

Vorzugsweise ist das mindestens eine anorganische Salz einer Peroxoschwefelsäure ausgewählt aus Ammoniumperoxodisulfat, Ammoniumperoxomonosulfat, Alkalimetallperoxodisulfaten, Alkalimetallperoxomonosulfaten und Alkalimetallhydrogenperoxomonosulfaten. Besonders bevorzugt sind Kaliumperoxodisulfat, Natriumperoxodisulfat, Ammoniumperoxodisulfat und Kaliumhydrogenperoxomonosulfat sowie Mischungen hiervon. Weiterhin hat es sich bei den Arbeiten zur vorliegenden Erfindung als besonders bevorzugt erwiesen, wenn das erfindungsgemäße oxidative Haaraufhellungsmittel mindestens zwei verschiedene Peroxodisulfate enthält. Bevorzugte Peroxodisulfatsalze sind dabei Kaliumperoxodisulfat-Ammoniumperoxodisulfat-Mischungen, Kaliumperoxodisulfat-Ammoniumperoxodisulfat-Natriumperoxodisulfat-Mischungen und Kaliumperoxodisulfat-Natriumperoxodisulfat-Mischungen. Besonders bevorzugt sind Mischungen aus Kaliumperoxodisulfat und Ammoniumperoxodisulfat, außerordentlich bevorzugt Mischungen aus Kaliumperoxodisulfat und Ammoniumperoxodisulfat mit einem Überschuss an Kaliumperoxodisulfat, die frei sind von Natriumperoxodisulfat. Erfindungsgemäß besonders bevorzugte Mischungen aus Kaliumperoxodisulfat (KPS) und Ammoniumperoxodisulfat (APS), die frei sind von Natriumperoxodisulfat, sind solche mit einem Gewichtsverhältnis KPS/APS, das im Bereich von 4:1 - 2:1, bevorzugt 3,5:1 - 2,5:1, besonders bevorzugt 3,2:1 - 2,8:1, liegt.

Bevorzugte erfindungsgemäße oxidative Haaraufhellungsmittel enthalten mindestens ein Oxidationsmittel, das aus anorganischen Salzen einer Peroxoschwefelsäure sowie Mischungen hiervon ausgewählt ist, in einer Gesamtmenge von 5 - 85 Gew.-%, bevorzugt 10 - 70 Gew.-%, besonders bevorzugt 17 - 55 Gew.-%, außerordentlich bevorzugt 30 - 45 Gew.-%, jeweils bezogen auf das Gewicht des oxidativen Haaraufhellungsmittels.

Das erfindungsgemäße oxidative Haaraufhellungsmittel enthält als zweiten wesentlichen Bestandteil Xanthan. Xanthan ist ein natürlich vorkommendes Polysaccharid, das mit Hilfe von Bakterien der Gattung Xanthomonas aus zuckerhaltigen Substraten gewonnen wird. Xanthan wirkt als hydrophiles Verdickungsmittel.

Erfindungsgemäß bevorzugte oxidative Haaraufhellungsmittel sind dadurch gekennzeichnet, dass Xanthan in einer Menge von 0,3 - 5 Gew.-%, bevorzugt 1 - 4 Gew.-%, besonders bevorzugt 1,5 - 3 Gew.-%, außerordentlich bevorzugt 2 - 2,5 Gew.-%, jeweils bezogen auf das Gewicht des oxidativen Haaraufhellungsmittels, enthalten ist.

Das erfindungsgemäße oxidative Haaraufhellungsmittel enthält als dritten wesentlichen Bestandteil ein Polysaccharid, das ausgewählt ist aus mikrokristalliner Cellulose und Mischungen von mikrokristalliner Cellulose und Guar Gum.

Mikrokristalline Cellulose, auch als Zusatzstoff E 460 i mit der CAS-Nummer 9004-34-6 bekannt, ist eine Cellulose-Modifikation, die als weißes, freifließendes Pulver vorliegt.

Guar Gum wird auch als Guar, Guaran oder Guarkernmehl bezeichnet. Guaran besteht aus D-Mannopyranose-Einheiten, die β-1,4-glycosidisch miteinander zu Ketten verknüpft sind. Zusätzlich trägt jede zweite Mannopyranoseeinheit über eine 1,6-Bindung α-D-Galactopyranosyl-Reste.

Erfindungsgemäß bevorzugte oxidative Haaraufhellungsmittel sind dadurch gekennzeichnet, dass mikrokristalline Cellulose in einer Menge von 0,01 - 5 Gew.-%, bevorzugt 0,02 - 2 Gew.-%, besonders bevorzugt 0,04 - 0,5 Gew.-%, außerordentlich bevorzugt 0,05 - 0,1 Gew.-%, jeweils bezogen auf das Gewicht des oxidativen Haaraufhellungsmittels, enthalten ist. Überraschend wurde festgestellt, dass ein geringer Zusatz an mikrokristalliner Cellulose zum erfindungsgemäß verwendeten Basisverdicker Xanthan die Anwendungseigenschaften des anwendungsbereiten Blondiermittels in überraschendem Ausmaß verbessert, insbesondere hinsichtlich einer Reduzierung des unerwünschten Quellens der Anwendungsmischung aus dem Haaraufhellungsmittel und einem Wasserstoffperoxid-haltigen Developer. Beim Einsatz von nicht-kristallinem Cellulose Gum anstelle der mikrokristallinen Cellulose werden die gewünschten Anwendungseigenschaften hingegen nicht erreicht.

Weitere erfindungsgemäß bevorzugte oxidative Haaraufhellungsmittel sind dadurch gekennzeichnet, dass mikrokristalline Cellulose in einer Menge von 0,01 - 5 Gew.-%, bevorzugt 0,02 - 2 Gew.-%, besonders bevorzugt 0,04 - 0,5 Gew.-%, außerordentlich bevorzugt 0,05 - 0,1 Gew.-%, jeweils bezogen auf das Gewicht des oxidativen Haaraufhellungsmittels, und Guar Gum in einer Menge von 0,01 - 5 Gew.-%, bevorzugt 0,02 - 2 Gew.-%, besonders bevorzugt 0,04 - 0,5 Gew.-%, außerordentlich bevorzugt 0,05 - 0,1 Gew.-%, jeweils bezogen auf das Gewicht des oxidativen Haaraufhellungsmittels, enthalten sind. Überraschend wurde festgestellt, dass ein geringer Zusatz an mikrokristalliner Cellulose und Guar Gum zum erfindungsgemäß verwendeten Basisverdicker Xanthan die Anwendungseigenschaften des anwendungsbereiten Blondiermittels in überraschendem Ausmaß verbessert, insbesondere hinsichtlich einer Reduzierung des unerwünschten Quellens der Anwendungsmischung aus dem Haaraufhellungsmittel und einem Wasserstoffperoxid-haltigen Developer in Verbindung mit einer Eindämmung des Temperaturanstiegs in der Anwendungsmischung.

Die erfindungsgemäßen oxidativen Haaraufhellungsmittel sind wasserfrei, was im Sinne der vorliegenden Erfindung bedeutet, dass sie, jeweils bezogen auf ihr Gewicht, 0 bis 10 Gew.-%, bevorzugt 0,1 bis 8 Gew.-%, besonders bevorzugt 0,5 bis 5 Gew.-% Wasser, enthalten.

Diese Angaben beziehen sich auf den Gehalt an freiem Wasser. Ein Gehalt an molekular gebundenem Wasser oder Kristallwasser, den einzelne Pulverbestandteile aufweisen können, wird nicht berücksichtigt. Der Wassergehalt kann beispielsweise in Anlehnung an ISO 4317 (Version 2011-12) mittels Karl-Fischer-Titration bestimmt werden.

Bestimmte Siliconverbindungen können die Schaumbildung unterdrücken, aber die vorliegenden Blondiermittel werden aus Gründen der ökologischen Nachhaltigkeit ohne Siliconverbindungen formuliert.

Erfindungsgemäß bevorzugte oxidative Haaraufhellungsmittel sind dadurch gekennzeichnet, dass mindestens einen Komplexbildner enthalten ist, der aus Komplexbildnern der allgemeinen Formel (I) ausgewählt ist: 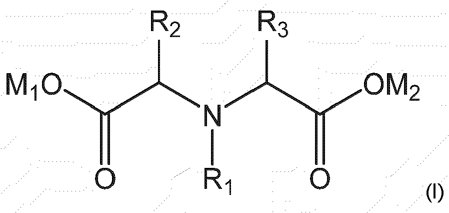 wobei
- R1: für ein Wasserstoffatom, eine C₁-C₆-Alkylgruppe, eine Hydroxy-C₂-C₆-alkylgruppe, eine Carboxy-C₁-C₆-Alkylgruppe oder für ein physiologisch verträgliches Salz hiervon steht,
- R2, R3: unabhängig voneinander für ein Wasserstoffatom, eine C₁-C₆-Alkylgruppe, eine Hydroxy-C₂-C₆-alkylgruppe, eine Carboxy-C₁-C₆-Alkylgruppe oder für ein physiologisch verträgliches Salz hiervon stehen, wobei R2 und R3 nicht gleichzeitig für ein Wasserstoffatom und nicht gleichzeitig für eine Carboxy-C₁-C₆-Alkylgruppe stehen,
- M1, M2: unabhängig voneinander für ein Wasserstoffatom oder ein Alkalimetallion, bevorzugt für Natrium, Kalium, oder für ein Ammoniumion (NH₄⁺) stehen.

Im Folgenden werden Beispiele für die in Formel (I) genannten Substituenten R1, R2 und R3 exemplarisch genannt, die erfindungsgemäß besonders geeignet sind. Beispiele für C₁-C₆-Alkylreste sind -CH₃, -CH₂CH₃, -CH₂CH₂CH₃, -CH(CH₃)₂, -CH₂CH(CH₃)₂, -CH(CH₃)CH₂CH₃, -C(CH₃)₃, -(CH₂)₄CH₃, -(CH₂)₅CH₃. Besonders bevorzugte C₁-C₆-Alkylreste sind Methyl und Ethyl; ein außerordentlich bevorzugter C₁-C₆-Alkylrest ist Methyl. Beispiele für C₁-C₆-Hydroxyalkylgruppen sind -CH₂OH, -CH₂CH₂OH, -CH₂CH₂CH₂OH, -CH₂CH(OH)CH₃, -CH₂CH₂CH₂CH₂OH, wobei -CH₂CH₂OH bevorzugt ist.

Beispiele für Carboxy-C₁-C₆-Alkylgruppen sind HOOC-CH₂-, HOOC-CH₂-CH₂-, HOOC-CH₂-CH₂-CH₂-, HOOC-CH₂-CH₂-CH₂-CH₂-, wobei die Gruppe HOOC-CH₂- erfindungsgemäß außerordentlich bevorzugt ist.

In den Komplexbildnern der allgemeinen Formel (I) steht der Rest R1 für ein Wasserstoffatom, eine C₁-C₆-Alkylgruppe, eine Hydroxy-C₂-C₆-alkylgruppe, eine Carboxy-C₁-C₆-Alkylgruppe oder für ein physiologisch verträgliches Salz einer Carboxy-C₁-C₆-Alkylgruppe.

Unter physiologisch verträglichen Salzen werden die Salze verstanden, die unter physiologischen Bedingungen ohne nachteilige Wirkung in Kosmetika eingesetzt werden können. Beispiele für ein physiologisch verträgliches Salz einer Carboxy-C₁-C₆-Alkylgruppe sind beispielsweise das Natriumsalz, das Kaliumsalz und das Ammoniumsalz der Carboxy-C₁-C₆-Alkylgruppe.

Eine Verbesserung hinsichtlich der verbesserten Kopfhautverträglichkeit und reduzierten Entwicklung von Mischungswärme konnte mit solchen erfindungsgemäßen Mitteln beobachtet werden, die mindestens einen Komplexbildner der allgemeinen Formel (I) enthielten, in der der Rest R1 für eine Carboxy-C₁-C₆-Alkylgruppe oder für ein physiologisch verträgliches Salz hiervon, bevorzugt für eine Carboxymethylgruppe oder für ein physiologisch verträgliches Salz hiervon, besonders bevorzugt für das Natriumsalz einer Carboxymethylgruppe, steht.

Im Rahmen einer besonders bevorzugten Ausführungsform ist ein erfindungsgemäßes Mittel dadurch gekennzeichnet, dass es mindestens einen Komplexbildner der allgemeinen Formel (I) enthält, wobei
- R1: für eine Carboxy-C₁-C₆-Alkylgruppe oder für ein physiologisch verträgliches Salz hiervon, bevorzugt für eine Carboxymethylgruppe oder für ein physiologisch verträgliches Salz hiervon, besonders bevorzugt für das Natriumsalz einer Carboxymethylgruppe, steht.

In den Komplexbildnern der allgemeinen Formel (I) stehen die Reste R2 und R3 unabhängig voneinander für ein Wasserstoffatom, eine C₁-C₆-Alkylgruppe, eine Hydroxy-C₂-C₆-alkylgruppe, eine Carboxy-C₁-C₆-Alkylgruppe oder für ein physiologisch verträgliches Salz hiervon, wobei R2 und R3 nicht gleichzeitig für jeweils ein Wasserstoffatom und nicht gleichzeitig für jeweils eine Carboxy-C₁-C₆-Alkylgruppe stehen.

In Blondierversuchen wurden des Weiteren besonders gute Ergebnisse bezüglich der Aufhellleistung und der reduzierten Wärmeentwicklung erzielt, wenn im erfindungsgemäßen Mittel mindestens ein Komplexbildner der Formel (I) eingesetzt wurde, bei welchem einer der Reste R2 oder R3 für ein Wasserstoffatom und der andere der Reste R2 oder R3 für eine Methylgruppe stehen.

Im Rahmen einer besonders bevorzugten Ausführungsform ist ein erfindungsgemäßes Mittel dadurch gekennzeichnet, dass es mindestens einen Komplexbildner der allgemeinen Formel (I) enthält, bei welchem einer der Reste R2 oder R3 für ein Wasserstoffatom und der andere der Reste R2 oder R3 für eine Methylgruppe stehen.

Die besten Ergebnisse bezüglich der Aufhellleistung und der reduzierten Wärmeentwicklung konnten mit Komplexbildnern der Formel (I) erhalten werden, bei welchen
- R1: für eine Carboxymethylgruppe oder für ein physiologisch verträgliches Salz hiervon, insbesondere für das Natriumsalz, steht,
- R2: für eine Methylgruppe steht und
- R3: für ein Wasserstoffatom steht.

Im Rahmen einer besonders bevorzugten Ausführungsform ist ein erfindungsgemäßes Mittel dadurch gekennzeichnet, dass es mindestens einen Komplexbildner der allgemeinen Formel (I) enthält, wobei
- R1: für eine Carboxymethylgruppe oder für ein Alkalimetall- oder ein Ammoniumsalz hiervon steht,
- R2: für eine Methylgruppe steht und
- R3: für ein Wasserstoffatom steht.

Ein explizit ganz besonders bevorzugter Komplexbildner dieser Ausführungsform ist N,N-Bis(carboxymethyl)-L-alanin, das auch als 2-Methyl-2',2",2‴-nitrilotriessigsäure bezeichnet werden kann und mit "MGDA" (Methylglycindiessigsäure) abgekürzt wird. MGDA hat die CAS-Nummer 29578-05-0. MGDA hat die Formel (I-a). Auch die Alkalimetallsalze und Ammoniumsalze von MGDA sind erfindungsgemäß bevorzugt. Besonders bevorzugt ist das Trinatriummethylglycindiacetat.

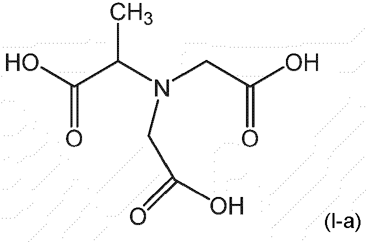

MGDA besitzt ein stereogenes Zentrum. Sowohl die D-Form als auch die L-Form und ein Gemisch aus D- und L-Form sind erfindungsgemäß.

Erfindungsgemäß besonders bevorzugte oxidative Haaraufhellungsmittel sind dadurch gekennzeichnet, dass der mindestens eine Komplexbildner, der aus Komplexbildnern der Formel (I) oder einem Alkalimetall- oder Ammoniumsalz dieser Säure ausgewählt ist, in einer Gesamtmenge von 0,1 bis 5,0 Gew.-%, bevorzugt 0,2 bis 2,0 Gew.-%, besonders bevorzugt 0,4 bis 1,6 Gew.-%, außerordentlich bevorzugt von 0,5 - 1,0 Gew.-%, jeweils bezogen auf das Gewicht des erfindungsgemäßen oxidativen Haaraufhellungsmittels, enthalten ist.

Weitere erfindungsgemäß besonders bevorzugte oxidative Haaraufhellungsmittel sind dadurch gekennzeichnet, dass der mindestens eine Komplexbildner, der aus Methylglycindiessigsäure und ihren Alkalimetall- oder Ammoniumsalzen ausgewählt ist, in einer Gesamtmenge von 0,1 bis 5,0 Gew.-%, bevorzugt 0,2 bis 2,0 Gew.-%, besonders bevorzugt 0,4 bis 1,6 Gew.-%, außerordentlich bevorzugt von 0,5 - 1,0 Gew.-%, jeweils bezogen auf das Gewicht des oxidativen Haaraufhellungsmittels, enthalten ist.

Weitere erfindungsgemäß besonders bevorzugte oxidative Haaraufhellungsmittel sind dadurch gekennzeichnet, dass der mindestens eine Komplexbildner, der aus den Natriumsalzen der Methylglycindiessigsäure ausgewählt ist, in einer Gesamtmenge von 0,1 bis 5,0 Gew.-%, bevorzugt 0,2 bis 2,0 Gew.-%, besonders bevorzugt 0,4 bis 1,6 Gew.-%, außerordentlich bevorzugt von 0,5 - 1,0 Gew.-%, jeweils bezogen auf das Gewicht des oxidativen Haaraufhellungsmittels, enthalten ist.

Weitere erfindungsgemäß besonders bevorzugte oxidative Haaraufhellungsmittel sind dadurch gekennzeichnet, dass das Trinatriumsalz der Methylglycindiessigsäure in einer Menge von 0,1 bis 5,0 Gew.-%, bevorzugt 0,2 bis 2,0 Gew.-%, besonders bevorzugt 0,4 bis 1,6 Gew.-%, außerordentlich bevorzugt von 0,5 - 1,0 Gew.-%, jeweils bezogen auf das Gewicht des oxidativen Haaraufhellungsmittels, enthalten ist.

Weitere erfindungsgemäß besonders bevorzugte oxidative Haaraufhellungsmittel sind durch einen Gehalt an Ammoniumchlorid gekennzeichnet. Überraschend wurde festgestellt, dass Ammoniumchlorid verschiedene Anwendungseigenschaften des erfindungsgemäßen oxidativen Haaraufhellungsmittels nach dem Vermischen mit einer wässrigen Wasserstoffperoxidzubereitung sehr günstig beeinflussen kann, darunter das Verhalten der Mischung sowie den sensorischen Eindruck der Mischung beim Antrocknen während der Einwirkzeit. Erfindungsgemäß bevorzugte oxidative Haaraufhellungsmittel sind dadurch gekennzeichnet, dass Ammoniumchlorid in einer Menge von 0,1 bis 10,0 Gew.-%, bevorzugt von 1,0 bis 8,0 Gew.-%, besonders bevorzugt von 2,0 bis 6,0 Gew.-%, außerordentlich bevorzugt von 3,1 - 5,0 Gew.-%, jeweils bezogen auf das Gewicht des oxidativen Haaraufhellungsmittels, enthalten ist.

Erfindungsgemäß besonders bevorzugte oxidative Haaraufhellungsmittel sind dadurch gekennzeichnet, dass sie mindestens eine Aminosäure, ausgewählt aus alpha-Aminosäuren, beta-Aminosäuren und den Salzen einer alpha-Aminosäure oder beta-Aminosäure, sowie Mischungen dieser Substanzen enthalten.

Unter einer Aminosäure ist eine chemische Verbindung mit einer Aminogruppe und einer Carbonsäuregruppe zu verstehen. Zur Klasse der Aminosäuren zählen organische Verbindungen, die zumindest eine Aminogruppe (-NH₂ bzw. substituiert -NR₂) und eine Carboxygruppe (-COOH) als funktionelle Gruppen enthalten, also Strukturmerkmale der Amine und der Carbonsäuren aufweisen. Chemisch lassen sie sich nach der Stellung ihrer Aminogruppe zur Carboxygruppe unterscheiden. Steht die Aminogruppe am Cₐₗₚₕₐ-Atom unmittelbar benachbart zur endständigen Carboxygruppe, nennt man dies alpha-ständig (α-ständig) und spricht von alpha-Aminosäuren (α-Aminosäuren). Steht die Aminogruppe am C_{beta}-Atom, von der endständigen Carboxygruppe aus gesehen, nennt man dies beta-ständig (β-ständig) und spricht von beta-Aminosäuren (β-Aminosäuren). Bevorzugt sind alpha-Aminosäuren und beta-Aminosäuren mit einer Gesamtzahl an C-Atomen von C2-C20, bevorzugter von C2-C15, besonders bevorzugt von C2-C10 enthalten.

Im Rahmen einer weiteren besonders bevorzugten Ausführungsform ist ein erfindungsgemäßes Mittel dadurch gekennzeichnet, dass es mindestens eine Aminosäure aus der Gruppe, bestehend aus Arginin, Serin, Histidin, Lysin, Asparagin, Glutamin, Cystein, Methionin, Tryptophan, Alanin, Asparaginsäure, Glutaminsäure, Glycin, Isoleucin, Leucin, Phenylalanin, Prolin, Threonin, Tyrosin, Valin und Taurin, den Salzen der vorgenannten Aminosäuren sowie Mischungen dieser Substanzen, enthält.

Erfindungsgemäß außerordentlich bevorzugte Aminosäuren sind ausgewählt aus der Gruppe, bestehend aus Arginin, Serin, den Salzen der vorgenannten Aminosäuren sowie Mischungen dieser Substanzen. Erfindungsgemäß außerordentlich bevorzugte Mittel sind dadurch gekennzeichnet, dass sie mindestens eine Aminosäure, ausgewählt aus der Gruppe, bestehend aus Arginin, Serin, den Salzen der vorgenannten Aminosäuren sowie Mischungen dieser Substanzen, enthalten.

Chirale Aminosäuren besitzen ein stereogenes Zentrum und können in spiegelbildlichen Formen auftreten. Beispielsweise tritt Arginin in Form des L-Arginins und des D-Arginins auf. Sowohl die L-Form einer Aminosäure als auch ihre D-Form sowie die Gemische hiervon sind von der vorliegenden Erfindung umfasst. Im Rahmen der vorliegenden Erfindung können demnach beide möglichen Enantiomere als spezifische Verbindung oder auch deren Gemische, insbesondere als Racemate, gleichermaßen eingesetzt werden. Es ist jedoch besonders vorteilhaft, die natürlicherweise vorkommende Isomerenform, üblicherweise die L-Konfiguration, einzusetzen.

Demzufolge ist ein besonders bevorzugtes ein erfindungsgemäßes Mittel dadurch gekennzeichnet, dass es mindestens eine Aminosäure aus der Gruppe aus L-Arginin, L-Serin, L-Histidin, L-Lysin, L-Asparagin, L-Glutamin, L-Cystein, L-Methionin, L-Tryptophan, L-Alanin, L-Asparaginsäure, L-Glutaminsäure, Glycin, L-Isoleucin, L-Leucin, L-Phenylalanin, L-Prolin, L-Threonin, L-Tyrosin und L-Valin und Taurin, den Salzen der vorgenannten Aminosäuren sowie Mischungen dieser Substanzen, enthält.

Weitere erfindungsgemäß besonders bevorzugte oxidative Haaraufhellungsmittel sind dadurch gekennzeichnet, dass die mindestens eine alpha-Aminosäure oder beta-Aminosäure und/oder ihr Salz in einer auf die Masse an freier Aminosäure umgerechneten Gesamtmenge von 0,1 bis 3,0 Gew.-%, bevorzugt 0,2 bis 1,5 Gew.-%, besonders bevorzugt 0,3 bis 0,6 Gew.-%, außerordentlich bevorzugt 0,4 - 0,5 Gew.-%, jeweils bezogen auf das Gewicht des oxidativen Haaraufhellungsmittels, enthalten ist.

Erfindungsgemäß außerordentlich bevorzugte oxidative Haaraufhellungsmittel enthalten mindestens eine alpha-Aminosäure, ausgewählt aus Arginin, Serin oder mindestens einem Salz dieser Aminosäuren, in einer auf die Masse an freier Aminosäure umgerechneten Gesamtmenge von 0,1 bis 3,0 Gew.-%, bevorzugt 0,2 bis 1,5 Gew.-%, besonders bevorzugt 0,3 bis 0,6 Gew.-%, außerordentlich bevorzugt 0,4 - 0,5 Gew.-%, jeweils bezogen auf das Gewicht des oxidativen Haaraufhellungsmittels.

Erfindungsgemäß außerordentlich bevorzugte oxidative Haaraufhellungsmittel enthalten mindestens eine Aminosäure, ausgewählt aus L-Arginin, L-Serin oder mindestens einem Salz dieser Aminosäuren, in einer auf die Masse an freier Aminosäure umgerechneten Gesamtmenge von 0,1 bis 3,0 Gew.-%, bevorzugt 0,2 bis 1,5 Gew.-%, besonders bevorzugt 0,3 bis 0,6 Gew.-%, außerordentlich bevorzugt 0,4 - 0,5 Gew.-%, jeweils bezogen auf das Gewicht des oxidativen Haaraufhellungsmittels.

Weitere erfindungsgemäß bevorzugte oxidative Haaraufhellungsmittel enthalten Polyquaternium-4. Bei Polyquaternium-4 handelt es sich um mit Diallyldimethylammoniumchlorid quaternisierte Hydroxyethylcellulose (INCI: Polyquaternium-4). Überraschend wurde festgestellt, dass Polyquaternium-4 das Aufquellverhalten erfindungsgemäß bevorzugter oxidativer Haaraufhellungsmittel nach dem Vermischen mit einer wässrigen Wasserstoffperoxidzubereitung weiter optimieren kann. Besonders bevorzugt ist Polyquaternium-4 in einer Gesamtmenge von 0,01 bis 3,0 Gew.-%, bevorzugt 0,1 bis 1,0 Gew.-%, besonders bevorzugt 0,1 bis 0,7 Gew.-%, außerordentlich bevorzugt 0,3 - 0,5 Gew.-%, jeweils bezogen auf das Gewicht des oxidativen Haaraufhellungsmittels, enthalten.

Für die Melanin abbauende Wirkung des Wasserstoffperoxids und die Blondierwirkung auf der keratinischen Faser ist es vorteilhaft, wenn die Anwendungsmischung aus Wasserstoffperoxid-Lösung und Persalz-haltigem Haaraufhellungsmittel einen alkalischen pH-Wert besitzt, der im Bereich von 8,0 bis 11,5, bevorzugt im Bereich von 8,5 bis 11,0, besonders bevorzugt 9,0 - 10,5, außerordentlich bevorzugt 9,5 - 10,3 liegt, jeweils gemessen bei 20°C.

Zur Einstellung eines alkalischen pH-Werts der aufhellenden Anwendungsmischung können erfindungsgemäß bevorzugte oxidative Haaraufhellungsmittel neben dem mindestens einen Persalz mindestens ein pulverförmiges Alkalisierungsmittel in einer solchen Gesamtmenge enthalten, dass die Anwendungsmischung aus erfindungsgemäßem oxidativen Haaraufhellungsmittel und Wasserstoffperoxid-Lösung den gewünschten alkalischen pH-Wert aufweist. Alternativ kann man der Anwendungsmischung aus erfindungsgemäßem oxidativen Haaraufhellungsmittel und Wasserstoffperoxid-Lösung eine dritte Zusammensetzung zusetzen, die ein oder mehrere Alkalisierungsmittel in einer solchen Menge enthält, dass die Anwendungsmischung einen alkalischen pH-Wert besitzt, der im Bereich von 8,0 bis 11,5, bevorzugt im Bereich von 8,5 bis 11,0, besonders bevorzugt 9,0 - 10,5, außerordentlich bevorzugt 9,5 - 10,3 liegt, jeweils gemessen bei 20°C.

Erfindungsgemäß bevorzugte oxidative Haaraufhellungsmittel sind daher dadurch gekennzeichnet, dass mindestens ein anorganisches, bei 20 °C und 1013 mbar (101.3 kPa) festes Alkalisierungsmittel enthalten ist. Erfindungsgemäß besonders bevorzugte oxidative Haaraufhellungsmittel enthalten mindestens ein anorganisches, bei 20 °C und 1013 mbar (101.3 kPa) festes Alkalisierungsmittel in einer Gesamtmenge von 4 - 70 Gew.-%, bevorzugt 10 - 60 Gew.-%, besonders bevorzugt 15 - 50 Gew.-%, weiterhin besonders bevorzugt 20 - 55 Gew.-%, außerordentlich bevorzugt 30 - 45 Gew.-%, jeweils bezogen auf das Gewicht des oxidativen Haaraufhellungsmittels.

Weitere erfindungsgemäß bevorzugte oxidative Haaraufhellungsmittel enthalten mindestens ein anorganisches, bei 20 °C und 1013 mbar (101.3 kPa) festes Alkalisierungsmittel, darunter in einer Gesamtmenge von 0,1 bis 50 Gew.-%, bevorzugt 4 bis 30 Gew.-%, besonders bevorzugt 10 - 15 Gew.-%, jeweils bezogen auf das Gewicht des oxidativen Haaraufhellungsmittels, mindestens ein Natriumsilikat oder Natriummetasilikat mit einem molaren SiO₂/Na₂O-Verhältnis von ≥ 2, bevorzugt 2,5-3,5.

Weitere erfindungsgemäß bevorzugte oxidative Haaraufhellungsmittel enthalten mindestens ein anorganisches, bei 20 °C und 1013 mbar (101.3 kPa) festes Alkalisierungsmittel, darunter in einer Gesamtmenge von 0,1 bis 20 Gew.-%, bevorzugt 1 bis 15 Gew.-%, besonders bevorzugt 3 - 10 Gew.-%, jeweils bezogen auf das Gewicht des oxidativen Haaraufhellungsmittels, mindestens ein Natriumsilikat oder Natriummetasilikat mit einem molaren SiO₂/Na₂O-Verhältnis von 0,9 - 1,1, bevorzugt 1.

Weitere erfindungsgemäß bevorzugte oxidative Haaraufhellungsmittel enthalten mindestens ein anorganisches, bei 20 °C und 1013 mbar (101.3 kPa) festes Alkalisierungsmittel, darunter in einer Gesamtmenge von 0,1 bis 40 Gew.-%, bevorzugt 1 bis 30 Gew.-%, besonders bevorzugt 5 - 25 Gew.-%, jeweils bezogen auf das Gewicht des oxidativen Haaraufhellungsmittels, mindestens ein Erdalkalimetallhydroxidcarbonat oder ein Erdalkalimetallcarbonat sowie Mischungen hiervon.

Erfindungsgemäß bevorzugte Erdalkalimetallhydroxidcarbonate sind Magnesiumhydroxidcarbonate mit der Formel MgCO₃ · Mg(OH)₂ · 2 H₂O und solche mit der Formel MgCO₃ · Mg(OH)₂. Weitere erfindungsgemäß bevorzugte oxidative Haaraufhellungsmittel enthalten mindestens ein anorganisches, bei 20 °C und 1013 mbar (101.3 kPa) festes Alkalisierungsmittel, darunter in einer Gesamtmenge von 0,1 bis 40 Gew.-%, bevorzugt 5 bis 35 Gew.-%, besonders bevorzugt 10 - 30 Gew.-%, jeweils bezogen auf das Gewicht des oxidativen Haaraufhellungsmittels, mindestens ein Magnesiumhydroxidcarbonat, das bevorzugt ausgewählt ist aus Magnesiumhydroxidcarbonaten mit der Formel MgCO₃ · Mg(OH)₂ · 2 H₂O und Magnesiumhydroxidcarbonaten mit der Formel MgCO₃ · Mg(OH)₂.

Als optionale Alkalisierungsmittel sind weitere erfindungsgemäß besonders bevorzugte anorganische, bei 20 °C und 1013 mbar (101.3 kPa) feste Alkalisierungsmittel ausgewählt aus Erdalkalimetallsilikaten, Erdalkalimetallmetasilikaten, Alkalimetallhydroxiden, Erdalkalimetallhydroxiden, (Erd-)Alkalimetallphosphaten und (Erd-)Alkalimetallhydrogenphosphaten sowie Mischungen dieser Substanzen.

Erfindungsgemäß besonders bevorzugte anorganische, bei 20°C und 1013 mbar (101.3 kPa) feste Alkalisierungsmittel sind Mischungen aus mindestens einem Natriumsilikat oder Natriummetasilikat mit einem molaren SiO₂/Na₂O-Verhältnis von 0,9 - 1,1, bevorzugt 1 und mindestens einem Magnesiumhydroxidcarbonat. Weitere Erfindungsgemäß besonders bevorzugte anorganische, bei 20°C und 1013 mbar (101.3 kPa) feste Alkalisierungsmittel sind Mischungen aus mindestens einem Natriumsilikat oder Natriummetasilikat mit einem molaren SiO₂/Na₂O-Verhältnis von 0,9 - 1,1, bevorzugt 1, mindestens einem Natriumsilikat oder Natriummetasilikat mit einem molaren SiO₂/Na₂O-Verhältnis von ≥ 2, bevorzugt 2,5-3,5 und mindestens einem Magnesiumhydroxidcarbonat.

Erfindungsgemäß besonders bevorzugte oxidative Haaraufhellungsmittel enthalten, jeweils bezogen auf ihr Gesamtgewicht, 0,1 bis 20 Gew.-%, bevorzugt 1 bis 15 Gew.-%, besonders bevorzugt 3 - 10 Gew.-% Natriumsilikate mit einem molaren SiO₂/Na₂O-Verhältnis von 0,9 - 1,1, bevorzugt 1, und 0,1 bis 40 Gew.-%, bevorzugt 5 bis 35 Gew.-%, besonders bevorzugt 10 - 30 Gew.-% Magnesiumhydroxidcarbonat als anorganische, bei 20 °C und 1013 mbar (101.3 kPa) feste Alkalisierungsmittel.

Erfindungsgemäß bevorzugte oxidative Haaraufhellungsmittel sind weiterhin dadurch gekennzeichnet, dass kein Polymer enthalten ist, das als Kettenbildendes Monomer Acrylsäure, Acrylsäureamide, Acrylsäureester, Methacrylsäure, Methacrylsäureamide, Methacrylsäureester, Vinylpyrrolidone, Vinylacetat, Vinylalkohol und/oder Styrole umfasst. Derartige Polymere können in bestimmten Fällen die Wärmeentwicklung im anwendungsbereiten Blondiermittel ungünstig beeinflussen; außerdem sind sie ökologisch weniger nachhaltig als natürliche Polymere.

Aus Sicherheitsgründen sollten die oxidativen Haaraufhellungsmittel, die als Blondierpulver konfektioniert sind, so formuliert werden, dass sie bei Gebrauch, insbesondere bevor oder während sie mit der flüssigen Wasserstoffperoxidlösung vermischt werden, nicht oder nur möglichst wenig Staub erzeugen. Geeignete Mittel zur Staubreduktion sind insbesondere Öle, die mit den Staub erzeugenden Bestandteilen des Blondierpulvers vermischt werden, wobei die Gesamtmenge an Ölen von 0,2 - 20 Gew.-%, bezogen auf das Gewicht des erfindungsgemäßen oxidativen Haaraufhellungsmittels, das als Blondierpulver vorliegt, beträgt.

Setzt man mehr als 20 Gew.-% Öl ein, so bildet sich mit den wasserfreien pulverförmigen Bestandteilen eine Paste. Erfindungsgemäße oxidative Haaraufhellungsmittel, die als Blondierpaste vorliegen, enthalten, jeweils bezogen auf ihr Gewicht, mindestens ein Öl in einer Gesamtmenge von 20,1 - 80 Gew.-%, bevorzugt 35 - 65 Gew.-%.

Durch geschickte Auswahl der Öle können dabei die Anwendungseigenschaften des oxidativen Haaraufhellungsmittels wie auch des gesamten anwendungsbereiten Aufhellungsmittels optimiert werden. Bei der Auswahl der Öle ist außerdem der Wunsch des Verbrauchers nach Produkten zu berücksichtigen, die möglichst wenig synthetisch hergestellte, sondern eher natürlich gewonnene Inhaltsstoffe enthalten. Öle mineralischer Herkunft, wie beispielsweise Mineralöle und Paraffine, zeigen gute Anwendungseigenschaften und sind preisgünstig verfügbar. Im Sinne der Nachhaltigkeit des gesamten Produkts können statt Ölen mineralischer Herkunft vorzugsweise Öle aus nachwachsenden Rohstoffquellen eingesetzt werden. Überraschend wurde festgestellt, dass bestimmte Öle das Aufquellverhalten des erfindungsgemäßen oxidativen Haaraufhellungsmittels nach dem Vermischen mit einer wässrigen Wasserstoffperoxidzubereitung sehr günstig beeinflussen können.

Erfindungsgemäß bevorzugte oxidative Haaraufhellungsmittel enthalten, bezogen auf ihr Gewicht, mindestens ein Öl in einer Gesamtmenge von 0,2 - 80 Gew.-%, bevorzugt 1 - 70 Gew.-%, besonders bevorzugt 3 - 60 Gew.-%, außerordentlich bevorzugt 4 - 20 Gew.-%.

Das mindestens eine Öl ist erfindungsgemäß bevorzugt ausgewählt aus Mineralölen, verzweigten Fettalkoholen mit 8 - 24 Kohlenstoffatomen, Dialkylethern mit 6 bis 18 C-Atomen in den Alkylgruppen, sowie Mischungen der vorgenannten Substanzen.

Erfindungsgemäß bevorzugte verzweigte Fettalkohole mit 8 - 24 Kohlenstoffatomen sind 2-Octyl-1-dodecanol, 2-Hexyl-1-decanol, 2-Ethyl-1-hexanol und Isostearylalkohol sowie Mischungen hiervon. Außerordentlich bevorzugt ist 2-Octyl-1-dodecanol.

Erfindungsgemäß bevorzugte Dialkylether mit 6 bis 18 C-Atomen in den Alkylgruppen sind Di-n-alkylether mit insgesamt 12 bis 36 C-Atomen, insbesondere 16 bis 24 C-Atomen im Molekül, wie beispielsweise Din-octylether, Di-n-decylether, Di-n-nonylether, Di-n-undecylether, Di-n-dodecylether, Di-n-octadecylether, n-Hexyl-n-octylether, n-Octyl-n-decylether, n-Decyl-n-undecylether, n-Undecyl-n-dodecylether und n-Hexyl-n-undecylether. Ebenfalls bevorzugte Dialkylether sind solche mit verzweigten Alkylgruppen mit jeweils 6 bis 18 C-Atomen, insbesondere mit Alkylgruppen, die in der 2-Position mit einer Ethylgruppe substituiert sind. Bevorzugte verzweigt-kettige Dialkylether mit 6 bis 18 C-Atomen in der Alkylgruppe sind ausgewählt aus Di-(2-ethylhexyl)ether und Di-(2-ethyldecyl)ether. Besonders bevorzugt ist Di-n-octylether (INCI: Dicaprylyl ether), der im Handel beispielsweise unter der Bezeichnung Cetiol^{®} OE von BASF erhältlich ist.

Erfindungsgemäß bevorzugte oxidative Haaraufhellungsmittel sind dadurch gekennzeichnet, dass sie, bezogen auf ihr Gewicht, mindestens ein Öl, ausgewählt aus Mineralölen, verzweigten Fettalkoholen mit 8 - 24 Kohlenstoffatomen, Dialkylethern mit 6 bis 18 C-Atomen in den Alkylgruppen, sowie Mischungen der vorgenannten Substanzen, in einer Gesamtmenge von 0,2 - 80 Gew.-%, bevorzugt 1 - 70 Gew.-%, besonders bevorzugt 3 - 60 Gew.-%, außerordentlich bevorzugt 4 - 20 Gew.-%, enthalten.

Erfindungsgemäß außerordentlich bevorzugte oxidative Haaraufhellungsmittel sind dadurch gekennzeichnet, dass sie, bezogen auf ihr Gewicht, mindestens ein Öl, ausgewählt aus Mineralölen, verzweigten Fettalkoholen mit 8 - 24 Kohlenstoffatomen, Dialkylethern mit 6 bis 18 C-Atomen in den Alkylgruppen, sowie Mischungen der vorgenannten Substanzen, in einer Gesamtmenge von 0,2 - 10 Gew.-%, bevorzugt 1 - 7 Gew.-%, besonders bevorzugt 3 - 6 Gew.-%, außerordentlich bevorzugt 4 - 5,5 Gew.-%, enthalten.

Weitere erfindungsgemäß außerordentlich bevorzugte oxidative Haaraufhellungsmittel sind dadurch gekennzeichnet, dass sie, bezogen auf ihr Gewicht, mindestens ein Öl, ausgewählt aus Mineralölen und 2-Octyl-1-dodecanol sowie Mischungen der vorgenannten Substanzen, in einer Gesamtmenge von 0,2 - 10 Gew.-%, bevorzugt 1 - 7 Gew.-%, besonders bevorzugt 3 - 6 Gew.-%, außerordentlich bevorzugt 4 - 5,5 Gew.-%, enthalten.

Weitere erfindungsgemäß außerordentlich bevorzugte oxidative Haaraufhellungsmittel sind dadurch gekennzeichnet, dass sie, bezogen auf ihr Gewicht, mindestens ein Öl, ausgewählt aus Mineralölen und Di-n-octylether sowie Mischungen der vorgenannten Substanzen, in einer Gesamtmenge von 0,2 - 10 Gew.-%, bevorzugt 1 - 7 Gew.-%, besonders bevorzugt 3 - 6 Gew.-%, außerordentlich bevorzugt 4 - 5,5 Gew.-%, enthalten.

Weiterhin sind auch Siliconöle erfindungsgemäß geeignete Öle. Esteröle, beispielsweise Benzoesäureester von linearen oder verzweigten C8-22-Alkanolen, wie Benzoesäure-C12-C15-alkylester, weiterhin Triglyceride (d. h. Dreifachester des Glycerins) von linearen oder verzweigten, gesättigten oder ungesättigten, gegebenenfalls hydroxylierten C8-30-Fettsäuren, bevorzugt gesättigte Triglyceridöle, insbesondere Capric/Caprylic Triglycerides, sowie natürliche Ölen, z.B. Amaranthsamenöl, Aprikosenkernöl, Arganöl, Avocadoöl, Babassuöl, Baumwollsaatöl, Borretschsamenöl, Distelöl, Erdnussöl, Granatapfelkernöl, Grapefruitsamenöl, Hanföl, Haselnussöl, Holundersamenöl, Johannisbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl und die flüssigen Anteile des Kokosöls, weiterhin den Dicarbonsäureester von linearen oder verzweigten C₂-C₁₀-Alkanolen, wie Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethyl-hexylsuccinat und Di-(2-hexyldecyl)-succinat, weiterhin Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, die bevorzugt ausgewählt sind aus bevorzugt 2-Hexyldecylstearat, 2-Hexyldecyllaurat, Isodecylneopentanoat, Isononylisononanoat, 2-Ethylhexylpalmitat und 2-Ethylhexylstearat, Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat, weiterhin C₈-C₂₂-Fettalkoholester einwertiger oder mehrwertiger C₂-C₇-Hydroxycarbonsäuren, insbesondere den Estern der Glycolsäure, Milchsäure, Äpfelsäure, Weinsäure, Citronensäure und Salicylsäure, z. B. C₁₂-C₁₅-Alkyllactat, weiterhin die symmetrischen, unsymmetrischen oder zyklischen Ester der Kohlensäure mit C₃₋₂₂-Alkanolen, C₃₋₂₂-Alkandiolen oder C₃₋₂₂-Alkantriolen, z. B. Dicaprylylcarbonat, können ebenfalls erfindungsgemäß als Öl eingesetzt werden, sind allerdings aufgrund der möglichen Gefahr einer Hydrolyse weniger bevorzugt bzw. sollten nur in geringen Mengen von 0,1 bis maximal 2 Gew.-%, bezogen auf das erfindungsgemäße oxidative Haaraufhellungsmittel, enthalten sein.

Weiterhin wurde festgestellt, dass ein Alkalimetall- oder Erdalkalimetallsalz einer C12-C24-Monocarbonsäure, die gesättigt oder ungesättigt, linear oder verzweigt sein kann, das Aufquellverhalten des erfindungsgemäßen oxidativen Haaraufhellungsmittels nach dem Vermischen mit einer wässrigen Wasserstoffperoxidzubereitung ungünstig beeinflussen kann.

Weitere erfindungsgemäß außerordentlich bevorzugte oxidative Haaraufhellungsmittel sind daher dadurch gekennzeichnet, dass kein Alkalimetall- oder Erdalkalimetallsalz einer C12-C24-Monocarbonsäure, die gesättigt oder ungesättigt, linear oder verzweigt sein kann, enthalten ist.

Erfindungsgemäß bevorzugte oxidative Haaraufhellungsmittel sind dadurch gekennzeichnet, dass kein Tensid und kein Emulgator enthalten sind. Tenside und Emulgatoren im Sinne der vorliegenden Anmeldung sind amphiphile (bifunktionelle) Verbindungen, die zur orientierten Absorption an Grenzflächen, zur Aggregation zu Mizellen und zur Ausbildung von lyotropen Phasen befähigt sind und die aus mindestens einem hydrophoben Molekülteil, der eine aliphatische Kohlenwasserstoffgruppe mit mindestens 8 Kohlenstoff-Atomen aufweist, und mindestens einem hydrophilen Molekülteil bestehen. Der hydrophobe Rest ist bevorzugt eine Kohlenwasserstoffkette mit 8-28 Kohlenstoff-Atomen, die gesättigt oder ungesättigt, linear oder verzweigt sein kann und nicht durch Heteroatome unterbrochen ist. Besonders bevorzugt ist diese C₈-C₂₈-Kohlenstoffkette linear.

Überraschend wurde festgestellt, dass Natriumhexametaphosphat das Aufquellverhalten des erfindungsgemäßen oxidativen Haaraufhellungsmittels nach dem Vermischen mit einer wässrigen Wasserstoffperoxidzubereitung sehr günstig beeinflussen kann. Weitere erfindungsgemäß außerordentlich bevorzugte oxidative Haaraufhellungsmittel sind daher dadurch gekennzeichnet, dass sie, jeweils bezogen auf ihr Gewicht, 0,05 - 0,5 Gew.-%, bevorzugt 0,2 - 0,4 Gew.-%, Natriumhexametaphosphat enthalten.

Überraschend wurde festgestellt, dass Kaolin verschiedene Anwendungseigenschaften des erfindungsgemäßen oxidativen Haaraufhellungsmittels nach dem Vermischen mit einer wässrigen Wasserstoffperoxidzubereitung sehr günstig beeinflussen kann, darunter das Schaumbildungs- und Aufquellverhalten, aber auch das Verhalten beim Antrocknen während der Einwirkzeit. Kaolin sorgt für eine cremige Textur der Anwendungsmischung. Darüber hinaus unterstützt Kaolin auf dem Haar die rasche Bildung eines trockenen Films auf der Anwendungsmischung. Weitere erfindungsgemäß außerordentlich bevorzugte oxidative Haaraufhellungsmittel sind daher dadurch gekennzeichnet, dass sie, jeweils bezogen auf ihr Gewicht, 0,1 - 20 Gew.-%, bevorzugt 1 - 15 Gew.-%, besonders bevorzugt 6 - 10 Gew.-% Kaolin enthalten.

In einer weiteren bevorzugten Ausführungsform enthält das erfindungsgemäße oxidative Haaraufhellungsmittel weiterhin mindestens eine Dicarbonsäure mit 2 bis 10 Kohlenstoffatomen, die besonders bevorzugt ausgewählt ist aus Bernsteinsäure, Oxalsäure, Malonsäure, Adipinsäure, Pimelinsäure, Korksäure, Azelainsäure, Sebacinsäure, Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure und/oder mindestens einem Salz dieser Säuren sowie Mischungen dieser Verbindungen, wobei die mindestens eine Dicarbonsäure mit 2 bis 10 Kohlenstoffatomen außerordentlich bevorzugt ausgewählt ist aus Bernsteinsäure, Äpfelsäure, Malonsäure und Maleinsäure sowie deren Salzen.

Erfindungsgemäß bevorzugte Salze der Dicarbonsäuren mit 2 bis 10 Kohlenstoffatomen sind ausgewählt aus den Mono- und Disalzen der Anionen von Bernsteinsäure, Oxalsäure, Malonsäure, Adipinsäure, Pimelinsäure, Korksäure, Azelainsäure, Sebacinsäure, Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure mit Alkalimetallionen, Erdalkalimetallionen und den Ionen von basischen Aminosäuren, wie Arginin, Lysin und Histidin, insbesondere mit Lithium-, Natrium-, Kalium-, Magnesium- und Calcium-Ionen, sowie Mischungen dieser Salze.

Die erfindungsgemäß besonders bevorzugte Bernsteinsäure weist bei 1013 mbar (101.3 kPa) einen Schmelzpunkt im Bereich von 185 - 187 °C auf, ist also bei 20°C ein Feststoff. Erfindungsgemäß geeignete Salze der Bernsteinsäure sind ausgewählt aus den Succinaten und Hydrogensuccinaten von Alkalimetallionen, Erdalkalimetallionen und den Ionen von basischen Aminosäuren, wie Arginin, Lysin und Histidin, insbesondere den Lithium-, Natrium-, Kalium-, Magnesium- und Calcium-Ionen, oder den Succinaten und Hydrogensuccinaten von basischen Aminosäuren, wie Arginin, Lysin und/oder Histidin, z.B. Argininsuccinat, sowie Mischungen dieser Salze. Die genannten Salze der Bernsteinsäure können auch gebundenes Kristallwasser enthalten, insbesondere das Natriumsuccinathexahydrat, das erfindungsgemäß besonders bevorzugt ist.

Die erfindungsgemäß besonders bevorzugte Äpfelsäure ist optisch aktiv. Aus Kostengründen ist racemische DL-Äpfelsäure bevorzugt. Erfindungsgemäß geeignete Salze der Äpfelsäure sind ausgewählt aus den Malaten und Hydrogenmalaten von Alkalimetallionen, Erdalkalimetallionen und den Ionen von basischen Aminosäuren, wie Arginin, Lysin und Histidin, insbesondere von Lithium-, Natrium-, Kalium-, Magnesium- und Calcium-Ionen, sowie Mischungen dieser Salze, insbesondere Dinatriummalat und Dikaliummalat, aber auch das Calciummalat. Die genannten, erfindungsgemäß geeigneten Salze der Äpfelsäure können gebundenes Kristallwasser enthalten, insbesondere das Dinatriummalathemihydrat und das Dinatriummalattrihydrat.

Erfindungsgemäß geeignete Salze der Oxalsäure sind ausgewählt aus den Oxalaten und Hydrogenoxalaten von Alkalimetallionen, Erdalkalimetallionen und den Ionen von basischen Aminosäuren, wie Arginin, Lysin und Histidin, insbesondere von Lithium-, Natrium-, Kalium-, Magnesium- und Calcium-Ionen, sowie Mischungen dieser Salze.

Erfindungsgemäß geeignete Salze der besonders bevorzugten Malonsäure sind ausgewählt aus den Malaten und Hydrogenmalaten von Alkalimetallionen, Erdalkalimetallionen und den Ionen von basischen Aminosäuren, wie Arginin, Lysin und Histidin, insbesondere von Lithium-, Natrium-, Kalium-, Magnesium- und Calcium-Ionen, sowie Mischungen dieser Salze.

Erfindungsgemäß geeignete Salze der Adipinsäure sind ausgewählt aus den Adipaten und Hydrogenadipaten von Alkalimetallionen, Erdalkalimetallionen und den Ionen von basischen Aminosäuren, wie Arginin, Lysin und Histidin, insbesondere von Lithium-, Natrium-, Kalium-, Magnesium- und Calcium-Ionen, sowie Mischungen dieser Salze.

Erfindungsgemäß geeignete Salze der Pimelinsäure sind ausgewählt aus den Pimelaten und Hydrogenpimelaten von Alkalimetallionen, Erdalkalimetallionen und den Ionen von basischen Aminosäuren, wie Arginin, Lysin und Histidin, insbesondere von Lithium-, Natrium-, Kalium-, Magnesium- und Calcium-Ionen, sowie Mischungen dieser Salze.

Erfindungsgemäß geeignete Salze der Korksäure sind ausgewählt aus den Suberaten und Hydrogensuberaten von Alkalimetallionen, Erdalkalimetallionen und den Ionen von basischen Aminosäuren, wie Arginin, Lysin und Histidin, insbesondere von Lithium-, Natrium-, Kalium-, Magnesium- und Calcium-Ionen, sowie Mischungen dieser Salze.

Erfindungsgemäß geeignete Salze der Azelainsäure sind ausgewählt aus den Azelaten und Hydrogenazelaten von Alkalimetallionen, Erdalkalimetallionen und den Ionen von basischen Aminosäuren, wie Arginin, Lysin und Histidin, insbesondere von Lithium-, Natrium-, Kalium-, Magnesium- und Calcium-Ionen, sowie Mischungen dieser Salze.

Erfindungsgemäß geeignete Salze der Sebacinsäure sind ausgewählt aus den Sebacaten und Hydrogensebacaten von Alkalimetallionen, Erdalkalimetallionen und den Ionen von basischen Aminosäuren, wie Arginin, Lysin und Histidin, insbesondere von Lithium-, Natrium-, Kalium-, Magnesium- und Calcium-Ionen, sowie Mischungen dieser Salze.

Erfindungsgemäß geeignete Salze der besonders bevorzugten Maleinsäure sind ausgewählt aus den Maleaten und Hydrogenmaleaten von Alkalimetallionen und Erdalkalimetallionen, insbesondere von Lithium-, Natrium-, Kalium-, Magnesium- und Calcium-Ionen, sowie Mischungen dieser Salze.

Erfindungsgemäß geeignete Salze der bevorzugten Fumarsäure sind ausgewählt aus den Fumaraten und Hydrogenfumaraten von Alkalimetallionen und Erdalkalimetallionen, insbesondere von Lithium-, Natrium-, Kalium-, Magnesium- und Calcium-Ionen, sowie Mischungen dieser Salze.

Erfindungsgemäß geeignete Salze der bevorzugten D-Weinsäure sind ausgewählt aus den Tartraten und Hydrogentartraten von Alkalimetallionen und Erdalkalimetallionen, insbesondere von Lithium-, Natrium-, Kalium-, Magnesium- und Calcium-Ionen, sowie Mischungen dieser Salze.

Erfindungsgemäß geeignete Salze der bevorzugten L-Weinsäure sind ausgewählt aus den Tartraten und Hydrogentartraten von Alkalimetallionen und Erdalkalimetallionen, insbesondere von Lithium-, Natrium-, Kalium-, Magnesium- und Calcium-Ionen, sowie Mischungen dieser Salze.

Erfindungsgemäß geeignete Salze der bevorzugten meso-Weinsäure sind ausgewählt aus den Tartraten und Hydrogentartraten von Alkalimetallionen und Erdalkalimetallionen, insbesondere von Lithium-, Natrium-, Kalium-, Magnesium- und Calcium-Ionen, sowie Mischungen dieser Salze.

Die erfindungsgemäß besonders bevorzugte Traubensäure ist das racemische Gemisch aus D-Weinsäure und L-Weinsäure. Erfindungsgemäß geeignete Salze der Traubensäure sind ausgewählt aus den Tartraten und Hydrogentartraten von Alkalimetallionen und Erdalkalimetallionen, insbesondere von Lithium-, Natrium-, Kalium-, Magnesium- und Calcium-Ionen, sowie Mischungen dieser Salze.

Erfindungsgemäß geeignete Salze der alpha-Ketoglutarsäure sind ausgewählt aus den alpha-Ketoglutaraten und alpha-Ketohydrogenglutaraten von Alkalimetallionen und Erdalkalimetallionen, insbesondere von Lithium-, Natrium-, Kalium-, Magnesium- und Calcium-Ionen, sowie Mischungen dieser Salze. Erfindungsgemäß geeignete Salze der beta-Ketoglutarsäure sind ausgewählt aus den beta-Ketoglutaraten und beta-Ketohydrogenglutaraten von Alkalimetallionen und Erdalkalimetallionen, insbesondere von Lithium-, Natrium-, Kalium-, Magnesium- und Calcium-Ionen, sowie Mischungen dieser Salze. Erfindungsgemäß geeignete Salze der Oxalessigsäure sind ausgewählt aus den Oxalacetaten und Oxalhydrogenacetaten von Alkalimetallionen und Erdalkalimetallionen, insbesondere von Lithium-, Natrium-, Kalium-, Magnesium- und Calcium-Ionen, sowie Mischungen dieser Salze.

Erfindungsgemäß bevorzugte oxidative Haaraufhellungsmittel enthalten die mindestens eine Dicarbonsäure mit 2 bis 10 Kohlenstoffatomen, die bevorzugt ausgewählt ist aus Bernsteinsäure, Oxalsäure, Malonsäure, Adipinsäure, Pimelinsäure, Korksäure, Azelainsäure, Sebacinsäure, Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure und/oder mindestens einem Salz dieser Säuren, in einer auf die Masse an freier Dicarbonsäure umgerechneten Gesamtmenge von 0,2 bis 4,0 Gew.-%, bevorzugt 0,33 bis 3,0 Gew.-%, besonders bevorzugt 0,4 bis 2,0 Gew.-%, außerordentlich bevorzugt 0,5 - 1,0 Gew.-%, jeweils bezogen auf das Gewicht des oxidativen Haaraufhellungsmittels.

Weitere erfindungsgemäß bevorzugte oxidative Haaraufhellungsmittel enthalten Bernsteinsäure und/oder mindestens ein Salz der Bernsteinsäure in einer auf die Masse an freier Dicarbonsäure umgerechneten Gesamtmenge von 0,2 bis 4,0 Gew.-%, bevorzugt 0,33 bis 3,0 Gew.-%, besonders bevorzugt 0,4 bis 2,0 Gew.-%, außerordentlich bevorzugt 0,5 - 1,0 Gew.-%, jeweils bezogen auf das Gewicht des oxidativen Haaraufhellungsmittels.

Weitere erfindungsgemäß bevorzugte oxidative Haaraufhellungsmittel enthalten Äpfelsäure und/oder mindestens ein Salz der Äpfelsäure in einer auf die Masse an freier Dicarbonsäure umgerechneten Gesamtmenge von 0,2 bis 4,0 Gew.-%, bevorzugt 0,33 bis 3,0 Gew.-%, besonders bevorzugt 0,4 bis 2,0 Gew.-%, außerordentlich bevorzugt 0,5 - 1,0 Gew.-%, jeweils bezogen auf das Gewicht des oxidativen Haaraufhellungsmittels.

Weitere erfindungsgemäß bevorzugte oxidative Haaraufhellungsmittel enthalten Malonsäure und/oder mindestens ein Salz der Malonsäure in einer auf die Masse an freier Dicarbonsäure umgerechneten Gesamtmenge von 0,2 bis 4,0 Gew.-%, bevorzugt 0,33 bis 3,0 Gew.-%, besonders bevorzugt 0,4 bis 2,0 Gew.-%, außerordentlich bevorzugt 0,5 - 1,0 Gew.-%, jeweils bezogen auf das Gewicht des oxidativen Haaraufhellungsmittels.

Überraschend wurde festgestellt, dass nichtionische Celluloseether, insbesondere nichtionische Celluloseether von C1-C4-Alkoholen, wie Methylcellulosen, Ethylcellulosen, Hydroxyalkylcellulosen (wie beispielsweise Hydroxyethylcellulose) und Methylhydroxyalkylcellulosen, sowie Mischungen von nichtionischen Celluloseethern und mikrokristalliner Cellulose im Gegensatz zu den erfindungsgemäßen Mischungen von Xanthan und mikrokristalliner Cellulose nicht annähernd die gewünschten Anwendungseigenschaften zeigten. Die nicht erfindungsgemäßen Verdickungsmittel reichten weder in der Konsistenz noch in Bezug auf die Krustenbildung an die erfindungsgemäßen Mischungen von Xanthan und mikrokristalliner Cellulose heran.

Erfindungsgemäß bevorzugte oxidative Haaraufhellungsmittel sind daher dadurch gekennzeichnet, dass sie keine nichtionischen Celluloseether enthalten.

Weitere erfindungsgemäß bevorzugte oxidative Haaraufhellungsmittel sind dadurch gekennzeichnet, dass sie, abgesehen von den obligatorischen Mischungen aus Xanthan und mikrokristalliner Cellulose oder Xanthan und mikrokristalline Cellulose und Guar Gum, weitere nichtionische oder anionische Polysaccharide entweder gar nicht oder nur in einer Gesamtmenge von 0,01 bis maximal 0,2 Gew.-%, bevorzugt 0,03 bis 0,1 Gew.-%, enthalten, jeweils bezogen auf das Gewicht des erfindungsgemäßen oxidativen Haaraufhellungsmittels. Es wurde festgestellt, dass insbesondere 0,01 - 0,2 Gew.-%, bevorzugt 0,03 bis 0,1 Gew.-%, an Cellulose Gum, bezogen auf das Gewicht des erfindungsgemäßen oxidativen Haaraufhellungsmittels, enthalten sein können, ohne die Anwendungseigenschaften des Blondiermittels zu beeinträchtigen.

Weiterhin können die erfindungsgemäßen oder erfindungsgemäß bevorzugten oxidativen Haaraufhellungsmittel mindestens einen direktziehenden Farbstoff enthalten. Dabei handelt es sich um Farbstoffe, die direkt auf das Haar aufziehen und keinen oxidativen Prozess zur Ausbildung der Farbe benötigen. Zur Mattierung von durch Melaninabbauprodukte hervorgerufene unerwünschte Restfarbeindrücke, insbesondere im rötlichen oder bläulichen Bereich, sind besonders bevorzugt bestimmte direktziehende Farbstoffe der komplementären Farben enthalten. Direktziehende Farbstoffe sind üblicherweise Nitrophenylendiamine, Nitroaminophenole, Azofarbstoffe, Anthrachinone oder Indophenole. Direktziehende Farbstoffe können anionisch, kationisch oder nichtionisch sein. Die direktziehenden Farbstoffe sind jeweils bevorzugt in einer Menge von 0,001 bis 2 Gew.-%, bezogen auf das Gewicht des oxidativen Haaraufhellungsmittels, enthalten.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterozyklus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Erfindungsgemäß ganz besonders bevorzugt ist eine Kombination aus Tetrabromphenolblau und Acid Red 92 enthalten.

Ein weiterer Gegenstand der vorliegenden Erfindung ist ein Verfahren zur Aufhellung oder zum aufhellenden Färben von Haaren, bei dem ein erfindungsgemäßes oder erfindungsgemäß bevorzugtes oxidatives Haaraufhellungsmittel (B) mit einer Oxidationszusammensetzung (Ox) vermischt wird, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und weiterhin mindestens ein pH-Stellmittel in einer solchen Menge enthält, dass die Oxidationszusammensetzung einen pH-Wert im Bereich von 1,5 bis 5,5, gemessen bei 20°C, aufweist, unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden, wobei das oxidative Haaraufhellungsmittel (B) und die Oxidationszusammensetzung (Ox) bevorzugt in einem gewichtsbezogenen Verhältnis (B) : (Ox) von 0,2 - 2, besonders bevorzugt 0,3 - 1,5, weiter bevorzugt 0,5 - 1, miteinander vermischt werden.

Die im erfindungsgemäßen Aufhellverfahren eingesetzte Oxidationszusammensetzung (Ox) enthält im Wesentlichen Wasser und Wasserstoffperoxid. Die Konzentration des Wasserstoffperoxids wird einerseits von den gesetzlichen Vorgaben und andererseits von dem gewünschten Effekt bestimmt. Sie beträgt 0,5 -20 Gew.-%, bevorzugt 3 - 12 Gew.-%, besonders bevorzugt 6 - 9 Gew.-% Wasserstoffperoxid (berechnet als 100%iges H₂O₂), jeweils bezogen auf das Gewicht der Oxidationszusammensetzung (Ox).

Die Oxidationszusammensetzung (Ox) besitzt zur Stabilisierung des Wasserstoffperoxids bevorzugt einen sauren pH-Wert, insbesondere einen pH-Wert im Bereich von 1,5 bis 5,5, gemessen bei 20°C. Zur Stabilisierung des Wasserstoffperoxids sind weiterhin bevorzugt Komplexbildner, Konservierungsmittel, Stabilisierungsmittel und/oder Puffersubstanzen enthalten.

Erfindungsgemäß bevorzugt ist das oxidative Haaraufhellungsmittel (B) so zusammengesetzt, dass die Mischung mit der vorgenannten Oxidationszusammensetzung (Ox), also das anwendungsbereite Aufhellungsmittel, insbesondere Blondiermittel, einen alkalischen pH-Wert, bevorzugt einen pH-Wert von 8,0 bis 11,5, bevorzugt im Bereich von 8,5 bis 11,0, besonders bevorzugt 9,0 - 10,5, außerordentlich bevorzugt 9,5 - 10,0, aufweist, jeweils gemessen bei 20 °C.

Erfindungsgemäß besonders bevorzugt verwendete Oxidationszusammensetzungen (Ox) enthalten weiterhin mindestens ein Öl und/oder mindestens eine Fettkomponente mit einem Schmelzpunkt im Bereich von 23 - 110 °C, bevorzugt in einer Gesamtmenge von 0,1 - 60 Gew.-%, besonders bevorzugt 0,5 - 40 Gew.-%, außerordentlich bevorzugt 2 - 24 Gew.-%, jeweils bezogen auf das Gewicht der erfindungsgemäß bevorzugt verwendeten Oxidationszusammensetzung (Ox).

Die für die erfindungsgemäß bevorzugt verwendeten Oxidationszusammensetzungen (Ox) geeigneten Öle sind zum großen Teil die gleichen Öle, die vorstehend als geeignete Entstaubungsmittel für Blondierpulver und als Trägeröle für Blondierpasten offenbart sind.

In den Oxidationszusammensetzungen (Ox) erfindungsgemäß bevorzugt verwendete Fettkomponenten mit einem Schmelzpunkt im Bereich von 23 - 110 °C sind ausgewählt aus linearen gesättigten 1-Alkanolen mit 12 - 30 Kohlenstoffatomen, bevorzugt in einer Gesamtmenge von 0,1 - 8 Gew.-%, besonders bevorzugt 3,0 bis 6,0 Gew.-%, jeweils bezogen auf das Gewicht der erfindungsgemäß verwendeten Oxidationszusammensetzung (Ox).

Bevorzugt ist das mindestens eine lineare gesättigte 1-Alkanol mit 12 - 30 Kohlenstoffatomen ausgewählt aus Laurylalkohol, Myristylalkohol, Cetylalkohol, Stearylalkohol, Arachidylalkohol, und Behenylalkohol sowie aus Mischungen dieser 1-Alkanole, besonders bevorzugt aus Cetylalkohol, Stearylalkohol und Cetylalkohol/ Stearylalkohol-Mischungen.

Erfindungsgemäß bevorzugt verwendete Oxidationszusammensetzungen (Ox) enthalten weiterhin, jeweils bezogen auf ihr Gewicht, mindestens ein lineares gesättigtes 1-Alkanol mit 12 - 30 Kohlenstoffatomen in einer Gesamtmenge von 0,1 - 8 Gew.-%, bevorzugt in einer Gesamtmenge von 2 - 6 Gew.-%, wobei mindestens ein 1-Alkanol, ausgewählt aus Cetylalkohol, Stearylalkohol und Cetylalkohol/Stearylalkohol-Mischungen, enthalten ist.

Weitere erfindungsgemäß bevorzugt verwendete Oxidationszusammensetzungen (Ox) enthalten mindestens eine Fettkomponente mit einem Schmelzpunkt im Bereich von 23 - 110 °C, die ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄ - C₃₆-Carbonsäuren sowie Mischungen der vorgenannten Substanzen. Weitere erfindungsgemäß bevorzugt verwendete Oxidationszusammensetzungen (Ox) enthalten mindestens ein Tensid oder mindestens einen Emulgator, bevorzugt in einer Gesamtmenge von 0,5 - 10 Gew.-%, bevorzugt 1 - 5 Gew.-%, jeweils bezogen auf das Gewicht der erfindungsgemäß verwendeten Oxidationszusammensetzung (Ox).

Tenside und Emulgatoren im Sinne der vorliegenden Anmeldung sind amphiphile (bifunktionelle) Verbindungen, die aus mindestens einem hydrophoben Molekülteil, der eine aliphatische Kohlenwasserstoffgruppe mit mindestens 8 Kohlenstoff-Atomen aufweist, und mindestens einem hydrophilen Molekülteil bestehen. Der hydrophobe Rest ist bevorzugt eine Kohlenwasserstoffkette mit 8-28 Kohlenstoff-Atomen, die gesättigt oder ungesättigt, linear oder verzweigt sein kann und nicht durch Heteroatome unterbrochen ist. Besonders bevorzugt ist diese C₈-C₂₈-Kohlenstoffkette linear. Basiseigenschaften der Tenside und Emulgatoren sind die orientierte Absorption an Grenzflächen sowie die Aggregation zu Mizellen und die Ausbildung von lyotropen Phasen.

Für den Einsatz in erfindungsgemäß bevorzugt verwendete Oxidationszusammensetzungen (Ox) sind anionische, nichtionische und kationische Tenside besonders geeignet. Aber auch zwitterionische und amphotere Tenside sind für den Einsatz in erfindungsgemäß bevorzugt verwendete Oxidationszusammensetzungen sehr geeignet.

Als anionische Tenside eignen sich in den erfindungsgemäß verwendeten Oxidationszusammensetzungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind lineare und verzweigte Fettsäuren mit 8 bis 30 C-Atomen (Seifen), Alkylethercarbonsäuren, Acylsarcoside, Acyltauride, Acylisethionate, Sulfobernsteinsäuremono-, -dialkylester und Sulfobernsteinsäuremono-alkylpolyoxyethylester, lineare Alkansulfonate, lineare alpha-Olefinsulfonate, Alkylsulfate und Alkylethersulfate sowie Alkyl- und/oder Alkenylphosphate. Bevorzugte anionische Tenside sind Alkylsulfate, Alkylethersulfate und Alkylethercarbonsäuren mit jeweils 10 bis 18 C-Atomen, bevorzugt 12 bis 14 C-Atomen in der Alkylgruppe und bis zu 12 Glykolethergruppen, bevorzugt 2 bis 6 Glykolethergruppen im Molekül. Beispiele solcher Tenside sind die Verbindungen mit den INCI-Bezeichnungen Sodium Laureth Sulfate, Sodium Lauryl Sulfate, Sodium Myreth Sulfate oder Sodium Laureth Carboxylate.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder SulfatGruppe tragen. Besonders geeignete zwitterionische Tenside sind die so genannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine - COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycin, N-Alkyltaurine, N-Alkylsarcosine, 2-Alkylaminopropionsäuren und Alkylaminoessigsäuren mit jeweils 8 bis 24 C-Atomen in der Alkylgruppe. Besonders bevorzugte amphotere Tenside sind N-Kokosalkylaminopropionat, Kokosacylaminoethylaminopropionat und C₁₂-C₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 4 bis 50 Mol Ethylenoxid und/oder 0 bis 5 Mol Propylenoxid an lineare und verzweigte Fettalkohole, an Fettsäuren und an Alkylphenole, jeweils mit 8 bis 20 C-Atomen in der Alkylgruppe, ethoxylierte Mono-, Di- und Triglyceride, wie beispielsweise Glycerinmonolaurat + 20 Ethylenoxid, und Glycerinmonostearat + 20 Ethylenoxid, Sorbitanfettsäureester und Anlagerungeprodukte von Ethylenoxid an Sorbitanfettsäureester wie beispielsweise die Polysorbate (Tween 20, Tween 21, Tween 60, Tween 61, Tween 81), Anlagerungsprodukte von Ethylenoxid an Fettsäurealkanolamide und Fettamine, sowie Alkylpolyglycoside. Als nichtionische Tenside eignen sich insbesondere C₈-C₂₂-Alkylmono- und -oligoglycoside und deren ethoxylierte Analoga sowie Ethylenoxid-Anlagerungsprodukte an gesättigte oder ungesättigte lineare Fettalkohole mit jeweils 2 bis 30 Mol Ethylenoxid pro Mol Fettalkohol. Weitere erfindungsgemäß bevorzugt verwendete Oxidationszusammensetzungen sind dadurch gekennzeichnet, dass das mindestens eine anionische Tensid ausgewählt ist aus Alkylsulfaten, Alkylethersulfaten und Alkylethercarbonsäuren mit jeweils 10 bis 18 C-Atomen, bevorzugt 12 bis 14 C-Atomen in der Alkylgruppe und bis zu 12, bevorzugt 2 bis 6 Glykolethergruppen, im Molekül.

Weitere erfindungsgemäß bevorzugt verwendete Oxidationszusammensetzungen sind dadurch gekennzeichnet, dass mindestens ein nichtionisches Tensid, ausgewählt aus Ethylenoxid-Anlagerungsprodukten an gesättigte oder ungesättigte lineare Fettalkohole mit jeweils 2 bis 30 Mol Ethylenoxid pro Mol Fettalkohol, sowie mindestens ein anionisches Tensid, ausgewählt aus Alkylsulfaten, Alkylethersulfaten und Alkylethercarbonsäuren mit jeweils 10 bis 18 C-Atomen, bevorzugt 12 bis 14 C-Atomen in der Alkylgruppe und bis zu 12, bevorzugt 2 bis 6 Glykolethergruppen, im Molekül, enthalten ist, wobei besonders bevorzugt das Gewichtsverhältnis der Gesamtheit aller anionischen Tenside zur Gesamtheit aller nichtionischen Tenside im Bereich von 5 - 50, bevorzugt 10 - 30 liegt.

Als kationische Tenside eignen sich in erfindungsgemäß bevorzugt verwendeten Oxidationszusammensetzungen (Ox) prinzipiell alle für die Verwendung am menschlichen Körper geeigneten kationischen oberflächenaktiven Stoffe. Diese sind gekennzeichnet durch mindestens eine wasserlöslich machende, kationische Gruppe, wie z. B. eine quaternäre Ammonium-Gruppe, oder durch mindestens eine wasserlöslich machende, kationisierbare Gruppe, wie z. B. eine Amin-Gruppe, und weiterhin mindestens eine (lipophil wirkende) Alkylgruppe mit 6 bis 30 C-Atomen oder mindestens eine (lipophil wirkende) Imidazol-Gruppe oder mindestens eine (lipophil wirkende) Imidazylalkyl-Gruppe.

Erfindungsgemäß besonders bevorzugt verwendete Oxidationszusammensetzungen (Ox) enthalten mindestens ein kationisches Tensid, das bevorzugt ausgewählt ist aus quarternären Ammoniumverbindungen mit mindestens einem C8-C24-Alkylrest, Esterquats und Amidoaminen mit jeweils mindestens einem C8-C24-Acylrest sowie Mischungen hiervon. Bevorzugte quaternäre Ammoniumverbindungen mit mindestens einem C8-C24-Alkylrest sind Ammoniumhalogenide, insbesondere Chloride, und Ammoniumalkylsulfate, wie Methosulfate oder Ethosulfate, wie C8-C24-Alkyltrimethylammoniumchloride, C8-C24-Dialkyldimethyl-ammoniumchloride und C8-C24-Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83, Quaternium-87 und Quaternium-91 bekannten Imidazolium-Verbindungen. Die Alkylketten der oben genannten Tenside weisen bevorzugt 8 bis 24 Kohlenstoffatome auf.

Bei Esterquats handelt es sich um kationische Tenside, die sowohl mindestens eine Esterfunktion als auch mindestens eine quartäre Ammoniumgruppe als Strukturelement und weiterhin mindestens eine C8-C24-Alkylrest oder C8-C24-Acylrest enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. N,N-Bis(2-Palmitoyloxy-ethyl)dimethylammoniumchlorid, Distearoylethyl Dimonium Methosulfate und Distearoylethyl Hydroxyethylmonium Methosulfate sind bevorzugte Beispiele für solche Esterquats.

Die Alkylamidoamine werden üblicherweise durch Amidierung natürlicher oder synthetischer C8-C24-Fettsäuren und Fettsäureschnitte mit Di-(C1-C3)alkylaminoaminen hergestellt. Eine erfindungsgemäß besonders geeignete Verbindung aus dieser Substanzgruppe ist Stearamidopropyldimethylamin. Erfindungsgemäß besonders bevorzugt verwendete Oxidationszusammensetzungen (Ox) enthalten mindestens ein kationisches Tensid in einer Gesamtmenge von 0,01 - 5 Gew.-%, bevorzugt 0,1 - 3 Gew.-%, besonders bevorzugt 0,3 - 2 Gew.-%, jeweils bezogen auf das Gewicht der erfindungsgemäß verwendeten Oxidationszusammensetzung (Ox).

Ein weiterer Gegenstand der vorliegenden Erfindung ist eine Mehrkomponentenverpackungseinheit (Kit-of-Parts) zur Aufhellung von keratinischen Fasern, die mindestens zwei getrennt voneinander verpackte Komponenten enthält und die dadurch gekennzeichnet ist, dass
i) die erste Komponente (B) ein erfindungsgemäßes oder ein erfindungsgemäß bevorzugtes oxidatives Haaraufhellungsmittel ist,
ii) die zweite Komponente (Ox) eine Oxidationszusammensetzung ist, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und einen pH-Wert im Bereich von 1,5 bis 5,5, gemessen bei 20°C, aufweist,
wobei die Komponenten (B) und (Ox) bevorzugt in einem gewichtsbezogenen Verhältnis (B) : (Ox) von 0,2 - 2, besonders bevorzugt 0,3 - 1,5, weiter bevorzugt 0,5 - 1, außerordentlich bevorzugt 0,5 - 0,6, zueinander vorliegen.

Die anwendungsbereiten Mischungen aus einem erfindungsgemäßen oder erfindungsgemäß bevorzugten oxidativen Haaraufhellungsmittel mit einer der vorgenannten Oxidationszusammensetzungen (Ox) weisen bevorzugt eine Viskosität im Bereich von 50.000 bis 160.000 mPas, besonders bevorzugt 80.000 bis 150.000 mPas auf, jeweils gemessen bei 20°C mit einem Brookfield-Viskosimeter, Spindel 6 mit einer Geschwindigkeit von 4 Umdrehungen/Minute. Eine Viskosität in diesem Bereich erlaubt, dass das anwendungsbereite Mittel sich einerseits gut auftragen lässt und andererseits über ein solches Fließverhalten verfügt, dass es für das Mittel eine ausreichend lange Einwirkzeit am Wirkort auf den keratinischen Fasern garantiert.

Eine Mehrkomponentenverpackungseinheit umfasst mehrere Einzelkomponenten, die getrennt voneinander konfektioniert sind, sowie eine gemeinsame Verpackung für diese Komponenten, zum Beispiel eine Faltschachtel. Darin werden die Komponenten jeweils getrennt in verschiedenen Containern bereitgestellt. Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder einer ähnlichen Umhüllung vorliegt. Dem Umhüllungsmaterial sind erfindungsgemäß keine Grenzen gesetzt. Bevorzugt handelt es sich jedoch dabei um Umhüllungen aus Glas oder Kunststoff.

Darüber hinaus kann die Verpackungseinheit Applikationshilfen, wie Kämme, Bürsten oder Pinsel, persönliche Schutzkleidung, insbesondere Einweg-Handschuhe, und eine Gebrauchsanleitung umfassen.

Bevorzugt beträgt die Einwirkzeit 5 bis 60 min, insbesondere 5 bis 50 min, besonders bevorzugt 10 bis 45 min. Während der Einwirkzeit der Mittel auf der Faser kann es vorteilhaft sein, den Aufhellvorgang durch Wärmezufuhr zu unterstützen. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Die Mittel ergeben bereits bei physiologisch verträglichen Temperaturen von unter 45°C gute Behandlungsergebnisse.

Nach Ende des Aufhellvorgangs werden alle auf den Keratinfasern befindlichen Komponenten mit Wasser oder einem tensidhaltigen Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellungsmittel einen höheren Tensidgehalt aufweist.

Das zu den erfindungsgemäßen und erfindungsgemäß bevorzugten oxidativen Haaraufhellungsmitteln Gesagte gilt mutatis mutandis auch für die erfindungsgemäßen und erfindungsgemäß bevorzugten Mehrkomponentenverpackungseinheiten (Kits of parts).

Das zu den erfindungsgemäßen und erfindungsgemäß bevorzugten oxidativen Haaraufhellungsmitteln Gesagte gilt mutatis mutandis auch für die erfindungsgemäßen und erfindungsgemäß bevorzugten Verfahren zur Aufhellung der keratinischen Fasern.

Das zu den erfindungsgemäß und erfindungsgemäß bevorzugt verwendeten Oxidationszusammensetzungen Gesagte gilt mutatis mutandis auch für die erfindungsgemäßen und erfindungsgemäß bevorzugten Mehrkomponentenverpackungseinheiten (Kits of parts).

Das zu den erfindungsgemäß und erfindungsgemäß bevorzugt verwendeten Oxidationszusammensetzungen Gesagte gilt mutatis mutandis auch für die erfindungsgemäßen und erfindungsgemäß bevorzugten Verfahren zur Aufhellung der keratinischen Fasern.

Das zu den erfindungsgemäßen und erfindungsgemäß bevorzugten oxidativen Haaraufhellungsmitteln Gesagte gilt mutatis mutandis auch für die erfindungsgemäße Verwendung.

Das zu den erfindungsgemäß und erfindungsgemäß bevorzugt verwendeten Oxidationszusammensetzungen Gesagte gilt mutatis mutandis auch für die erfindungsgemäße Verwendung.

Die nachfolgenden Beispiele stellen den Gegenstand der Erfindung dar, ohne ihn hierauf zu beschränken. Experimenteller Teil

### 1. Untersuchungen zum Aufquellverhalten

**Tabelle 1: Blondierpulver (alle Mengenangaben in Gew.-%)**

| | 01\01 (V1) | 01\02 (V2) | 01 \03 (erfindungsgemäß E1) | 01\04 (V3) |
|---|---|---|---|---|
| wasserfreies Natriummetasilikat mit einem molaren SiO₂/Na₂O-Verhältnis von 0,9 - 1,1 | 3,0 | 3,0 | 3,0 | 3,0 |
| Hydrous sodium silicate mit einem molaren SiO₂/Na₂O-Verhältnis von 2,65 (Britesil C 265 von Akzo) | 13,0 | 13,0 | 13,0 | 13,0 |
| basisches Magnesiumcarbonat | 31,35 | 28,85 | 28,77 | 28,77 |
| Natriumhexametaphosphat | 0,4 | 0,4 | 0,4 | 0,4 |
| Xanthan Gum | - | 2,5 | 2,5 | 2,5 |
| mikrokristalline Cellulose | - | - | 0,08 | - |
| Cellulose Gum | - | - | - | 0,08 |
| Kaolin | 10,0 | 10,0 | 10,0 | 10,0 |
| Trinatriummethylglycindiacetat (Na₃-MGDA) | 0,45 | 0,45 | 0,45 | 0,45 |
| L-Arginin | 0,2 | 0,2 | 0,2 | 0,2 |
| L-Serin | 0,2 | 0,2 | 0,2 | 0,2 |
| Bernsteinsäure | 0,6 | 0,6 | 0,6 | 0,6 |
| Ammoniumpersulfat (mit 0,5 Gew.-% Silica) | 8,0 | 8,0 | 8,0 | 8,0 |
| Kaliumpersulfat | 28,0 | 28,0 | 28,0 | 28,0 |
| Paraffinum liquidum | 4,8 | 4,8 | 4,8 | 4,8 |
| | 100,0 | 100,0 | 100,0 | 100,0 |

**Tabelle 2: Oxidationsmittel-haltiger Entwickler**

| **Inhaltsstoff** | **Einwaage (Gew.-%)** |
|---|---|
| Natriumbenzoat | 0,04 |
| Dipicolinsäure (2,6-Dicarboxypyridin) | 0,10 |
| Dinatriumpyrophosphat | 0,10 |
| Kaliumhydroxid | 0,12 |
| Etidronsäure | 0,15 |
| Paraffinöl | 17,00 |
| Natriumcetearylsulfat | 0,36 |
| Cetearylalkohol | 3,50 |
| PEG-40 Castor Oil | 0,70 |
| Wasserstoffperoxid | 9,00 |
| Wasser | ad 100,00 |

Jedes der Blondierpulver aus Tabelle 1 wurde mit der Wasserstoffperoxid-haltigen Öl-in-Wasser-Emulsion gemäß Tabelle 2 im Gewichtsverhältnis 1:1 in einer Anmischschale vermischt und in einen Messzylinder gefüllt. Der zeitliche Verlauf des Volumens der anwendungsbereiten Blondiermittelmischung wurde in regelmäßigen Zeitabständen gemessen. Die Porengröße des Schaums wurde optisch mit bloßem Auge durch geschultes Fachpersonal beurteilt. Die Ergebnisse stehen in Tabelle 3:

**Tabelle 3: zeitlicher Verlauf des Volumens der anwendungsbereiten Blondiermittelmischung [ml]**

| Zeit t [Minuten nach Füllen der Mischung in Messzylinder] | V1 | V2 | E1 | V3 |
|---|---|---|---|---|
| 0 | 48 | 48 | 48 | 48 |
| 3 | 54 | 54 | 53 | 60 |
| 5 | 58 | 58 | 58 | 65 [1] |
| 10 | 68 | 67 | 66 | 77 [2] |
| 15 | 76 [1] | 77 [1] | 78 [1] | 88 [3] |
| 20 | 85 [2] | 84 [1] | 90 [2] | 99 [3] |
| 30 | 107 [3] | 105 [2] | 112 [2] | 123 [3] |
| 35 | 117 [3] | 115 [2] | 124 [3] | 134 [3] |
| 40 | 127 [3] | 125 [2] | 135 [3] | 144 [3] |
| 45 | 136 [3] | 134 [2] | 145 [3] | 154 [3] |
| | | | | |

| relative Zunahme des Volumens | | | | |
|---|---|---|---|---|
| nach 15 Minuten | 158% | 160% | 163% | 183% |
| nach 30 Minuten | 223% | 219% | 233% | 256% |
| nach 45 Minuten | 283% | 279% | 302% | 321% |

Die Porigkeit des Schaums wird in drei Kategorien unterteilt: [1] feinporig, [2] mittelporig und [3] grobporig.

### 2. Untersuchungen zur Verdickung und Krustenbildung

**Tabelle 4: Oxidationsmittel-haltiger Entwickler für Blondierpulver**

| **Inhaltsstoff** | **Einwaage (Gew.-%)** |
|---|---|
| Natriumbenzoat | 0,04 |
| Dipicolinsäure (2,6-Dicarboxypyridin) | 0,10 |
| Dinatriumpyrophosphat | 0,10 |
| Kaliumhydroxid | 0,12 |
| Etidronsäure | 0,15 |
| Paraffinöl | 17,00 |
| Natriumcetearylsulfat | 0,36 |
| Cetearylalkohol | 3,50 |
| **PEG-40** Castor Oil | 0,70 |
| Wasserstoffperoxid | 6,00 |
| Wasser | ad 100,00 |

Jedes der Blondierpulver aus Tabelle 1 wurde mit der Wasserstoffperoxid-haltigen Öl-in-Wasser-Emulsion gemäß Tabelle 4 im Gewichtsverhältnis 1:1,5 (2 Gewichtsteile Blondierpulver und 3 Gewichtsteile H₂O₂-Entwickler) in einer Anmischschale zu einem anwendungsbereiten Blondiermittel vermischt und auf trockene Haarsträhnen vom Farbton 6-0 (light brown, Fischbach & Miller) aufgetragen.

Von geschultem Fachpersonal wurde mit bloßem Auge die Konsistenz der Anwendungsmischung in der Schale [S] und auf der Haarsträhne [HS] beurteilt. Die Ergebnisse stehen in Tabelle 5:

**Tabelle 5: Konsistenz der Anwendungsmischung in der Schale [S] und auf der Haarsträhne [HS]**

| Zeit t [Minuten nach dem Vermischen] | V1 | V2 | E1 | V3 |
|---|---|---|---|---|
| Start | [S]: cremig, glatt, wie dünne Nivea-Creme | [S]: cremig, krümelig, wie dicke Nivea | [S]: cremig, leicht krümelig, wie dicke Nivea | [S]: cremig, leicht krümelig, wie dicke Nivea |
| nach 10' | [S]: cremig, nass, wie dünne Nivea | [S]: cremig, nass, wie dicke Nivea | [S]: cremig, nass, wie dicke Nivea | [S]: cremig, nass, wie dicke Nivea |
| | [HS]: feucht, klebrig | [HS]: feucht, klebrig | [HS]: feucht, klebrig | [HS]: feucht, klebrig |
| nach 20' | [S]: cremig, nass, wie dünne Nivea | [S]: cremig, klebrig, nass | [S]: cremig, klebrig, | [S]: cremig, klebrig, nass, leicht quillt |
| | [HS]: feucht, klebrig | [HS]: feucht, klebrig | **[HS]: klebrige trockene Kruste** | [HS]: feucht, klebrig, quillt leicht |
| nach 30' | [S]: cremig, nass, quillt leicht, | [S]: cremig, klebrig | [S]: cremig, **trockener Film** | [S]: cremig, klebrig, quillt |
| | [HS]: feucht, klebrig | [HS]: feucht, klebrig | **[HS]: trockene Kruste** | **[HS]: klebrige Kruste**, quillt |
| nach 45' | [S]: sandig, nass, quillt | [S]: cremig, klebrig, quillt | **[S]: trockener Film** | [S]: trockener Film, quillt |
| | [HS]: feucht klebrig, quillt leicht | [HS]: feucht, klebrig, quillt leicht | **[HS]: trockene Kruste,** quillt leicht | **[HS]: trockene Kruste,** quillt |
| pH-Wert der Anwendungsmischung | 10,0 | 10,0 | 10,0 | 10,1 |
| Viskosität der Anwendungsmischung [Pa·s] | 45 | 149 | 138 | 172 |

Nur die Anwendungsmischung mit dem erfindungsgemäßen Blondierpulver zeigte die gewünschten Anwendungseigenschaften, nämlich eine cremige, kompakte Konsistenz in der Schale und die Ausbildung einer Kruste, die die Schaumbildung des Mittels während der Einwirkzeit limitiert, dadurch einen besseren Kontakt des Blondiermittels mit der Keratinfaser während der gesamten Einwirkzeit garantiert und so das Aufhellungsergebnis verbessert. Durch die kompakte Konsistenz und die Ausbildung einer Kruste wird außerdem die Gefahr verringert, dass die Anwendungsmischung auf die Kopfhaut fließt und dort Irritationen verursacht.

Die Viskosität der Anwendungsmischung wurde mit bevorzugt mittels Brookfield-Viskosimeter, Spindel Nr. 6, 4 Umdrehungen pro Minute, bei 20°C, bestimmt.

### 3. Untersuchungen zur Blondierleistung bzw. zum Aufhellvermögen

Jedes der Blondierpulver aus Tabelle 1 wurde mit der Wasserstoffperoxid-haltigen Öl-in-Wasser-Emulsion gemäß Tabelle 4 im Gewichtsverhältnis 1:1,5 (2 Gewichtsteile Blondierpulver und 3 Gewichtsteile H₂O₂-Entwickler) in einer Anmischschale zu einem anwendungsbereiten Blondiermittel vermischt und auf trockene Haarsträhnen vom Farbton 6-0 (light brown, Fischbach & Miller) aufgetragen (Flottenverhältnis von 4 Gramm anwendungsbereites Blondiermittel pro Gramm Haar) und nach 45 Minuten Einwirkzeit wieder ausgespült. Die anwendungsbereiten Blondiermittel wiesen jeweils einen pH-Wert von 10,0 bzw. 10,1 (V3) auf, gemessen bei 20°C.

Die blondierten Strähnen wurden nach dem Waschen und Trocknen farbmetrisch nach dem L a b- System vermessen. Die farbmetrischen Werte sind in Tabelle 6 zusammengestellt.

**Tabelle 6: Farbmetrische Werte**

| | V1 | V2 | E1 | V3 | 6-0 Strähnen ungebleicht |
|---|---|---|---|---|---|
| L | 57,19 | 57,70 | 58,52 | 56,39 | 25,42 |
| a | 10,54 | 10,13 | 9,58 | 10,31 | 6,17 |
| b | 32,80 | 31,91 | 31,85 | 32,44 | 10,07 |
| | | | | | |
| dL | 31,77 | 32,28 | 33,10 | 30,97 | |
| da | 4,37 | 3,96 | 3,41 | 4,14 | |
| db | 22,73 | 21,84 | 21,78 | 22,37 | |
| dE | 39,31 | 39,17 | 39,77 | 38,43 | |
| | | | | | |
| Levels of Lift (Aufhellstufen) | 7,9 | 7,8 | 8,0 | 7,7 | |

### Interpretation der farbmetrischen Werte aus Tabelle 6:

Je größer die Werte für L bzw. dL sind, desto stärker aufgehellt ist die Strähne.

Mit dem erfindungsgemäßen Blondierpulver wurde der höchste Grad an Aufhellung erzielt.

Die Farbdifferenz zwischen ungebleichter und gebleichter Strähne, für die jeweils eine L*,a*,b*-Wertekombination bestimmt wurde, wurde gemäß folgender Formel berechnet:$dE = {\left({dL}^{2} + {da}^{2} + {db}^{2}\right)}^{0,5}$

Je größer der Wert für dE ist, desto stärker ausgeprägt ist die Farbdifferenz.

Mit dem erfindungsgemäßen Blondierpulver wurde die höchste Farbdifferenz erzielt.

Das erfindungsgemäße Blondierpulver wies weiterhin den geringsten a-Wert auf. Die erfindungsgemäß erzielte Aufhellung war damit auf der grün-rot-Achse stärker in den kühleren Grün-Bereich verschoben als bei den Vergleichspulvern, die zum wärmeren Rot tendierten.

Das erfindungsgemäße Blondierpulver wies weiterhin den geringsten b-Wert auf. Die erfindungsgemäß erzielte Aufhellung war damit auf der blau-gelb-Achse stärker in den kühleren Blau-Bereich verschoben als bei den Vergleichspulvern, die zum wärmeren Gelb tendierten.

### 4. Vergleich des Aufquellverhaltens von Xanthan Gum und Hydroxyethylcellulose

**Tabelle 7: Blondierpulver (alle Mengenangaben in Gew.-%)**

| | V4 | V5 | E2 | V6 |
|---|---|---|---|---|
| wasserfreies Natriummetasilikat mit einem molaren SiO₂/Na₂O-Verhältnis von 0,9 - 1,1 | 3,00 | 3,00 | 3,00 | 3,00 |
| Hydrous sodium silicate mit einem molaren SiO₂/Na₂O-Verhältnis von 2,65 (Britesil C 265 von Akzo) | 13,00 | 13,00 | 13,00 | 13,00 |
| basisches Magnesiumcarbonat (Magnesiumhydroxidcarbonat) | 28,95 | 28,95 | 28,87 | 28,87 |
| Natriumhexametaphosphat | 0,40 | 0,40 | 0,40 | 0,40 |
| Hydroxyethylcellulose | - | 2,50 | - | 2,50 |
| Xanthan Gum | 2,50 | - | 2,50 | - |
| mikrokristalline Cellulose | - | - | 0,08 | 0,08 |
| Kaolin | 10,00 | 10,00 | 10,00 | 10,00 |
| Trinatriummethylglycindiacetat (Na₃-MGDA) | 0,45 | 0,45 | 0,45 | 0,45 |
| L-Arginin | 0,20 | 0,20 | 0,20 | 0,20 |
| L-Serin | 0,20 | 0,20 | 0,20 | 0,20 |
| Polyquaternium-4 | 0,30 | 0,30 | 0,30 | 0,30 |
| Bernsteinsäure | 0,60 | 0,60 | 0,60 | 0,60 |
| Ammoniumpersulfat (mit 0,5 Gew.-% Silica) | 8,00 | 8,00 | 8,00 | 8,00 |
| Kaliumpersulfat | 28,00 | 28,00 | 28,00 | 28,00 |
| Paraffinum Liquidum | 4,40 | 4,40 | 4,40 | 4,40 |
| | 100,00 | 100,00 | 100,00 | 100,00 |

Jedes der Blondierpulver aus Tabelle 7 wurde mit der Wasserstoffperoxid-haltigen Öl-in-Wasser-Emulsion gemäß Tabelle 2 im Gewichtsverhältnis 1:1 in einer Anmischschale vermischt und in einen Messzylinder gefüllt. Der zeitliche Verlauf des Volumens der anwendungsbereiten Blondiermittelmischung wurde in regelmäßigen Zeitabständen gemessen. Die Ergebnisse stehen in Tabelle 8:

**Tabelle 8: zeitlicher Verlauf des Volumens der anwendungsbereiten Blondiermittelmischung [ml]**

| Zeit t [Minuten nach Füllen der Mischung in Messzylinder] | V4 | V5 | E2 | V6 |
|---|---|---|---|---|
| 0 | 52 | 54 | 52 | 54 |
| 3 | 55 | 58 | 56 | 58 |
| 5 | 64 | 66 | 60 | 64 |
| 10 | 80 | 74 | 74 | 76 |
| 15 | 98 | 84 | 82 | 88 |
| 20 | 114 | 95 | 98 | 104 |
| 30 | 145 | 118 | 115 | 128 |
| 35 | 160 | 128 | 126 | 140 |
| 40 | 175 | 138 | 138 | 152 |
| 45 | 186 | 148 | 147 | 162 |
| | | | | |

| relative Zunahme des Volumens | | | | |
|---|---|---|---|---|
| nach 15 Minuten | 188% | 156% | 158% | 163% |
| nach 30 Minuten | 279% | 219% | 221% | 237% |
| nach 45 Minuten | 358% | 274% | 283% | 300% |

Der Vergleich der beiden nicht erfindungsgemäßen Blondierpulver V4 und V5 miteinander zeigt, dass in Abwesenheit von mikrokristalliner Cellulose der nichtionische Celluloseether Hydroxyethylcellulose als alleiniges Verdickungsmittel das Aufquellvolumen stärker reduziert, als wenn Xanthan als alleiniges Verdickungsmittel enthalten ist. Allerdings zeigen Blondierpulver mit Hydroxyethylcellulose als alleinigem Verdickungsmittel nicht die gewünschten Eigenschaften bei der Krustenbildung.

Der Vergleich des erfindungsgemäßen Blondierpulvers E2 mit dem nicht erfindungsgemäßen Blondierpulver V6 zeigt, dass in Gegenwart von mikrokristalliner Cellulose mit Xanthan als Verdickungsmittel das Volumen stärker reduziert wird als mit der Kombination von mikrokristalliner Cellulose und Hydroxyethylcellulose.

### 5. Weitere Untersuchungen zum Aufquellverhalten und zur Wärmetönung

**Tabelle 9: Blondierpulver (alle Mengenangaben in Gew.-%)**

| | E4 | V4 | E3 (nicht erfindungsgemäß) | E2 |
|---|---|---|---|---|
| wasserfreies Natriummetasilikat mit einem molaren SiO₂/Na₂O-Verhältnis von 0,9 - 1,1 | 3,00 | 3,00 | 3,00 | 3,00 |
| Hydrous sodium silicate mit einem molaren SiO₂/Na₂O-Verhältnis von 2,65 (Britesil C 265 von Akzo) | 13,00 | 13,00 | 13,00 | 13,00 |
| basisches Magnesiumcarbonat (Magnesiumhydroxidcarbonat) | 28,79 | 28,95 | 28,87 | 28,87 |
| Natriumhexametaphosphat | 0,40 | 0,40 | 0,40 | 0,40 |
| Guar Gum | 0,08 | - | 0,08 | - |
| Xanthan Gum | 2,50 | 2,50 | 2,50 | 2,50 |
| mikrokristalline Cellulose | 0,08 | - | - | 0,08 |
| Kaolin | 10,00 | 10,00 | 10,00 | 10,00 |
| Trinatriummethylglycindiacetat (Na₃-MGDA) | 0,45 | 0,45 | 0,45 | 0,45 |
| L-Arginin | 0,20 | 0,20 | 0,20 | 0,20 |
| L-Serin | 0,20 | 0,20 | 0,20 | 0,20 |
| Polyquaternium-4 | 0,30 | 0,30 | 0,30 | 0,30 |
| Bernsteinsäure | 0,60 | 0,60 | 0,60 | 0,60 |
| Ammoniumpersulfat (mit 0,5 Gew.-% Silica) | 8,00 | 8,00 | 8,00 | 8,00 |
| Kaliumpersulfat | 28,00 | 28,00 | 28,00 | 28,00 |
| Paraffinum Liquidum | 4,40 | 4,40 | 4,40 | 4,40 |
| | 100,00 | 100,00 | 100,00 | 100,00 |

Jedes der Blondierpulver aus Tabelle 9 wurde mit der Wasserstoffperoxid-haltigen Öl-in-Wasser-Emulsion gemäß Tabelle 2 im Gewichtsverhältnis 1:1 in einer Anmischschale vermischt und in einen Messzylinder gefüllt. Der zeitliche Verlauf des Volumens der anwendungsbereiten Blondiermittelmischung wurde in regelmäßigen Zeitabständen gemessen. Die Ergebnisse stehen in Tabelle 10:

**Tabelle 10: zeitlicher Verlauf des Volumens der anwendungsbereiten Blondiermittelmischung [ml]**

| Zeit t [Minuten nach Füllen der Mischung in Messzylinder] | E4 | V4 | E3 (nicht erfindungsgemäß) | E2 |
|---|---|---|---|---|
| 0 | 52 | 52 | 52 | 52 |
| 3 | 54 | 55 | 58 | 56 |
| 5 | 58 | 64 | 66 | 60 |
| 10 | 72 | 80 | 78 | 74 |
| 15 | 85 | 98 | 92 | 82 |
| 20 | 95 | 114 | 108 | 98 |
| 30 | 120 | 145 | 135 | 115 |
| 35 | 130 | 160 | 148 | 126 |
| 40 | 140 | 175 | 164 | 138 |
| 45 | 146 | 186 | 174 | 147 |
| | | | | |

| relative Zunahme des Volumens | | | | |
|---|---|---|---|---|
| nach 15 Minuten | 163% | 188% | 177% | 158% |
| nach 30 Minuten | 231% | 279% | 260% | 221% |
| nach 45 Minuten | 281% | 358% | 335% | 283% |

Der Vergleich der erfindungsgemäßen Blondierpulver E2 und E4 mit dem nicht-erfindungsgemäßen Blondierpulver V4 zeigt, dass der Zusatz von mikrokristalliner Cellulose zum Basis-Verdickungsmittel Xanthan das Aufquellvolumen der anwendungsbereiten Blondiermittelmischung reduziert.

Wenn, wie im erfindungsgemäßen Blondierpulver E4, sowohl mikrokristalline Cellulose als auch Guar Gum zum Basis-Verdickungsmittel Xanthan zugesetzt werden, bringt dies zwar keine weitere Verbesserung bei der Volumenreduzierung, aber die Dreier-Kombination aus Xanthan, mikrokristalliner Cellulose und Guar führt zu einer verbesserten Wärmetönung der anwendungsbereiten Blondiermittelmischung, das heißt zu einer geringeren Temperatur in der Mischung, siehe Tabelle 11.

**Tabelle 11: zeitlicher Verlauf der Temperatur der anwendungsbereiten Blondiermittelmischung (Temperaturangaben in °C)**

| Zeit t [Minuten nach Herstellung der anwendungsbereiten Blondiermittelmischung] | E4 | V4 | E3 (nicht erfindungsgemäß) | E2 |
|---|---|---|---|---|
| 0 | 20,7 | 20,4 | 22,1 | 22,1 |
| 1 | 22,4 | 22,2 | 23,0 | 23,0 |
| 2 | 22,9 | 22,7 | 23,4 | 23,3 |
| 3 | 23,2 | 23,0 | 23,6 | 23,5 |
| 5 | 23,5 | 23,4 | 24,1 | 24,0 |
| 10 | 24,1 | 24,3 | 24,6 | 24,8 |
| 15 | 24,3 | 24,9 | 25,0 | 25,4 |
| 20 | 24,5 | 25,5 | 25,4 | 25,9 |
| 25 | 24,6 | 26,1 | 25,7 | 26,4 |
| 30 | 24,7 | 26,6 | 25,9 | 26,8 |
| 35 | 24,8 | 26,9 | 26,1 | 27,1 |
| 40 | 24,8 | 27,2 | 26,3 | 27,4 |
| 45 | 24,8 | 27,5 | 26,4 | 27,6 |

Jedes der Blondierpulver aus Tabelle 9 wurde mit der Wasserstoffperoxid-haltigen Öl-in-Wasser-Emulsion gemäß Tabelle 2 im Gewichtsverhältnis 1:1 in einer Anmischschale vermischt. Die Temperatur der anwendungsbereiten Blondiermittelmischung wurde in regelmäßigen Zeitabständen gemessen. Die Ergebnisse sind in Tabelle 11 dargestellt.

### 6. Untersuchungen zur Blondierleistung

**Tabelle 12: Oxidationsmittel-haltiger Entwickler**

| **Inhaltsstoff** | **Einwaage (Gew.-%)** |
|---|---|
| Natriumbenzoat | 0,04 |
| Dipicolinsäure (2,6-Dicarboxypyridin) | 0,10 |
| Dinatriumpyrophosphat | 0,10 |
| Kaliumhydroxid | 0,12 |
| Etidronsäure | 0,15 |
| Paraffinöl | 17,00 |
| Natriumcetearylsulfat | 0,36 |
| Cetearylalkohol | 3,50 |
| PEG-40 Castor Oil | 0,70 |
| Wasserstoffperoxid | 6,00 |
| Wasser | ad 100,00 |

Jedes der Blondierpulver aus Tabelle 9 wurde mit der Wasserstoffperoxid-haltigen Öl-in-Wasser-Emulsion gemäß Tabelle 12 im Gewichtsverhältnis 1:1,5 in einer Anmischschale vermischt. 100 g dieser frisch hergestellten Mischung aus dem Blondierpulver und der Entwickleremulsion wurden auf trockene Haarsträhnen (mittelbraun (Kerling ENH 4/0)) appliziert (4 g Anwendungsmischung pro Gramm Haar). Nachdem die Strähnen 45 Minuten lang blondiert wurden, wurden sie 2 Minuten lang mit Wasser ausgewaschen und mit einem Föhn getrocknet. Anschließend wurden die Strähnen farbmetrisch vermessen. Die farbmetrischen Werte sind in Tabelle 13 zusammengestellt.

**Tabelle 13: farbmetrische Werte**

| Kerling ENH 4/0 | dL | da | db | dE | Levels of Lift |
|---|---|---|---|---|---|
| E4 | 25,53 | 8,15 | 24,40 | 36,24 | 7,2 |
| V4 | 22,73 | 7,54 | 21,77 | 32,36 | 6,5 |
| E3 (nicht erfindungsgemäß) | 21,39 | 7,27 | 19,95 | 30,14 | 6,0 |
| E2 | 23,48 | 7,37 | 21,97 | 32,99 | 6,6 |

Größere L-Werte und größere Farbabstandswerte dE sind gleichbedeutend mit einer stärkeren Aufhellung.

Der Vergleich der erfindungsgemäßen Blondierpulver E2 und E4 mit dem nicht-erfindungsgemäßen Blondierpulver V4 zeigt, dass der Zusatz von mikrokristalliner Cellulose zum Basis-Verdickungsmittel Xanthan die Blondierleistung der anwendungsbereiten Blondiermittelmischung leicht verbessert.

Wenn, wie im erfindungsgemäßen Blondierpulver E4, sowohl mikrokristalline Cellulose als auch Guar Gum zum Basis-Verdickungsmittel Xanthan zugesetzt werden, führt das zu einer deutlich verbesserten Blondierleistung der anwendungsbereiten Blondiermittelmischung, siehe Tabelle 13.

## Patentansprüche

1. Oxidatives Haaraufhellungsmittel zum Aufhellen oder zum aufhellenden Färben der Haare, das in nicht-komprimierter Pulverform oder als fließfähige Paste vorliegt, enthaltend
a) mindestens ein Oxidationsmittel, ausgewählt aus den anorganischen Salzen einer Peroxoschwefelsäure, sowie Mischungen dieser Salze,
b) Xanthan,
c) ein Polysaccharid, ausgewählt aus mikrokristalliner Cellulose und Mischungen von mikrokristalliner Cellulose und Guar Gum,
d) und 0 bis 10 Gew.-%, bevorzugt 0,1 bis 8 Gew.-%, besonders bevorzugt 0,5 bis 5 Gew.-% Wasser, jeweils bezogen auf das Gewicht des oxidativen Haaraufhellungsmittels,
e) wobei keine Siliconverbindungen enthalten sind.

2. Oxidatives Haaraufhellungsmittel gemäß Anspruch 1, **dadurch gekennzeichnet, dass** Xanthan in einer Menge von 0,3 - 5 Gew.-%, bevorzugt 1 - 4 Gew.-%, besonders bevorzugt 1,5 - 3 Gew.-%, außerordentlich bevorzugt 2 - 2,5 Gew.-%, jeweils bezogen auf das Gewicht des oxidativen Haaraufhellungsmittels, enthalten ist.

3. Oxidatives Haaraufhellungsmittel gemäß Anspruch 1 oder 2, **dadurch gekennzeichnet, dass** mikrokristalline Cellulose in einer Menge von 0,01 - 5 Gew.-%, bevorzugt 0,02 - 2 Gew.-%, besonders bevorzugt 0,04 - 0,5 Gew.-%, außerordentlich bevorzugt 0,05 - 0,1 Gew.-%, jeweils bezogen auf das Gewicht des oxidativen Haaraufhellungsmittels, enthalten ist.

4. Oxidatives Haaraufhellungsmittel gemäß einem der Ansprüche 1 - 3, **dadurch gekennzeichnet, dass** Guar Gum in einer Menge von 0,01 - 5 Gew.-%, bevorzugt 0,02 - 2 Gew.-%, besonders bevorzugt 0,04 - 0,5 Gew.-%, außerordentlich bevorzugt 0,05 - 0,1 Gew.-%, jeweils bezogen auf das Gewicht des oxidativen Haaraufhellungsmittels, enthalten ist.

5. Oxidatives Haaraufhellungsmittel gemäß einem der Ansprüche 1 - 4, **dadurch gekennzeichnet, dass** das mindestens eine Oxidationsmittel a) ausgewählt ist aus Natriumperoxodisulfat, Kaliumperoxodisulfat, Ammoniumperoxodisulfat, Natriumperoxomonosulfat, Kaliumperoxomonosulfat, Ammoniumperoxomonosulfat sowie Mischungen dieser Verbindungen, bevorzugt Kaliumperoxodisulfat-Ammoniumperoxodisulfat-Mischungen, Kaliumperoxodisulfat-Ammoniumperoxodisulfat-Natriumperoxodisulfat-Mischungen und Kaliumperoxodisulfat-Natriumperoxodisulfat-Mischungen, besonders bevorzugt aus Kaliumperoxodisulfat-Ammoniumperoxodisulfat-Mischungen, außerordentlich bevorzugt aus Kaliumperoxodisulfat-Ammoniumperoxodisulfat-Mischungen mit einem Überschuss an Kaliumperoxodisulfat, die frei sind von Natriumperoxodisulfat.

6. Oxidatives Haaraufhellungsmittel gemäß einem der Ansprüche 1 - 5, **dadurch gekennzeichnet, dass** das mindestens eine Oxidationsmittel a) in einer Gesamtmenge von 5 - 85 Gew.-%, bevorzugt 10 - 70 Gew.-%, besonders bevorzugt 17 - 55 Gew.-%, außerordentlich bevorzugt 30 - 45 Gew.-%, jeweils bezogen auf das Gewicht des oxidativen Haaraufhellungsmittels, enthalten ist.

7. Oxidatives Haaraufhellungsmittel gemäß einem der Ansprüche 1 - 6, **dadurch gekennzeichnet, dass** weiterhin mindestens ein Komplexbildner enthalten ist, ausgewählt aus Komplexbildnern der allgemeinen Formel (I), 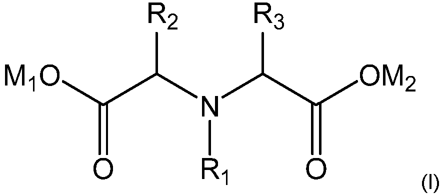 wobei
R1 für ein Wasserstoffatom, eine C₁-C₆-Alkylgruppe, eine Hydroxy-C₂-C₆-alkylgruppe, eine Carboxy-C₁-C₆-Alkylgruppe oder für ein physiologisch verträgliches Salz hiervon steht,
R2, R3 unabhängig voneinander für ein Wasserstoffatom, eine C₁-C₆-Alkylgruppe, eine Hydroxy-C₂-C₆-alkylgruppe, eine Carboxy-C₁-C₆-Alkylgruppe oder für ein physiologisch verträgliches Salz hiervon stehen, wobei R2 und R3 nicht gleichzeitig für ein Wasserstoffatom und nicht gleichzeitig für eine Carboxy-C₁-C₆-Alkylgruppe stehen,
M1, M2 unabhängig voneinander für ein Wasserstoffatom oder ein Alkalimetallion, bevorzugt für Natrium, Kalium, oder für ein Ammoniumion (NH₄⁺) stehen.

8. Oxidatives Haaraufhellungsmittel gemäß Anspruch 7, **dadurch gekennzeichnet, dass** der mindestens eine Komplexbildner der allgemeinen Formel (I) ausgewählt ist aus Methylglycindiessigsäure, den Natriumsalzen, Kaliumsalzen und Ammoniumsalzen der Methylglycindiessigsäure sowie Mischungen der vorgenannten Substanzen, wobei das Dinatriumsalz und das Trinatriumsalz der Methylglycindiessigsäure bevorzugt sind und das Trinatriumsalz der Methylglycindiessigsäure besonders bevorzugt ist.

9. Oxidatives Haaraufhellungsmittel gemäß einem der Ansprüche 7 oder 8, **dadurch gekennzeichnet, dass** der mindestens eine Komplexbildner der allgemeinen Formel (I) in einer Gesamtmenge von 0,1 bis 5,0 Gew.-%, bevorzugt 0,2 bis 2,0 Gew.-%, besonders bevorzugt 0,4 bis 1,6 Gew.-%, außerordentlich bevorzugt von 0,5 - 1,0 Gew.-%, jeweils bezogen auf das Gewicht des oxidativen Haaraufhellungsmittels, enthalten ist.

10. Oxidatives Haaraufhellungsmittel gemäß einem der Ansprüche 7 - 9, **dadurch gekennzeichnet, dass** das Trinatriumsalz der Methylglycindiessigsäure in einer Menge von 0,1 bis 5,0 Gew.-%, bevorzugt 0,2 bis 2,0 Gew.-%, besonders bevorzugt 0,4 bis 1,6 Gew.-%, außerordentlich bevorzugt von 0,5 - 1,0 Gew.-%, jeweils bezogen auf das Gewicht des oxidativen Haaraufhellungsmittels, enthalten ist.

11. Oxidatives Haaraufhellungsmittel gemäß einem der Ansprüche 1 - 10, **dadurch gekennzeichnet, dass** weiterhin Ammoniumchlorid enthalten ist, bevorzugt in einer Menge von 0,1 bis 10,0 Gew.-%, bevorzugt 1,0 bis 8,0 Gew.-%, besonders bevorzugt 2,0 bis 6,0 Gew.-%, außerordentlich bevorzugt von 3,1 - 5,0 Gew.-%, jeweils bezogen auf das Gewicht des oxidativen Haaraufhellungsmittels.

12. Oxidatives Haaraufhellungsmittel gemäß einem der Ansprüche 1 - 11, **dadurch gekennzeichnet, dass** 0,05 - 0,5 Gew.-%, bevorzugt 0,2 - 0,4 Gew.-% Natriumhexametaphosphat, jeweils bezogen auf das Gewicht des oxidativen Haaraufhellungsmittels, enthalten ist.

13. Oxidatives Haaraufhellungsmittel gemäß einem der Ansprüche 1 - 12, **dadurch gekennzeichnet, dass** mindestens ein anorganisches, bei 20 °C und 1013 mbar (101.3 kPa) festes Alkalisierungsmittel in einer Gesamtmenge von 4 - 70 Gew.-%, bevorzugt 10 - 60 Gew.-%, besonders bevorzugt 15 - 50 Gew.-%, weiterhin besonders bevorzugt 20 - 55 Gew.-%, außerordentlich bevorzugt 30 - 45 Gew.-%, jeweils bezogen auf das Gewicht des oxidativen Haaraufhellungsmittels, jeweils bezogen auf das Gewicht des oxidativen Haaraufhellungsmittels, enthalten ist.

14. Verfahren zur Aufhellung oder zum aufhellenden Färben von keratinischen Fasern, insbesondere menschlichen Haaren, **dadurch gekennzeichnet, dass** ein oxidatives Haaraufhellungsmittel (B) nach einem der Ansprüche 1 - 13 mit einer Oxidationszusammensetzung (Ox) vermischt wird, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid enthält und weiterhin mindestens ein pH-Stellmittel in einer solchen Menge enthält, dass die Oxidationszusammensetzung einen pH-Wert im Bereich von 1,5 bis 5,5, gemessen bei 20°C, aufweist, unmittelbar danach auf die keratinhaltigen Fasern aufgebracht, 5 bis 60 Minuten auf den Fasern belassen und anschließend die Fasern mit Wasser gespült und optional mit einem tensidhaltigen Reinigungsmittel ausgewaschen werden, wobei das oxidative Haaraufhellungsmittel (B) und die Oxidationszusammensetzung (Ox) bevorzugt in einem gewichtsbezogenen Verhältnis (B) : (Ox) von 0,2 - 2, besonders bevorzugt 0,3 - 1,5, weiter bevorzugt 0,5 - 1, miteinander vermischt werden.

15. Mehrkomponentenverpackungseinheit (Kit-of-Parts) zur Aufhellung von keratinischen Fasern, die mindestens zwei getrennt voneinander verpackte Komponenten enthält und die **dadurch gekennzeichnet ist, dass**
i) die erste Komponente (B) ein oxidatives Haarbehandlungsmittel nach einem der Ansprüche 1 - 13 ist,
ii) die zweite Komponente (Ox) eine Oxidationszusammensetzung ist, die, jeweils bezogen auf ihr Gewicht, 50 - 96 Gew.-%, bevorzugt 70 - 93 Gew.-%, besonders bevorzugt 80 - 90 Gew.-%, Wasser und 0,5 - 20 Gew.-% Wasserstoffperoxid und weiterhin mindestens ein pH-Stellmittel in einer solchen Menge enthält, dass die Oxidationszusammensetzung einen pH-Wert im Bereich von 1,5 bis 5,5, gemessen bei 20°C, aufweist,
wobei die Komponenten (B) und (Ox) bevorzugt in einem gewichtsbezogenen Verhältnis (B) : (Ox) von 0,2 - 2, besonders bevorzugt 0,3 - 1,5, weiter bevorzugt 0,5 - 1, außerordentlich bevorzugt 0,5 - 0,6, zueinander vorliegen.

## Claims

1. An oxidative hair lightening agent for lightening hair or coloring hair lighter, in the form of a non-compressed powder form or a flowable paste, containing
a) at least one oxidizing agent selected from inorganic salts of a peroxysulfuric acid, and mixtures of these salts,
b) xanthan gum,
c) a polysaccharide selected from microcrystalline cellulose and mixtures of microcrystalline cellulose and guar gum,
d) and 0 to 10 wt.%, preferably 0.1 to 8 wt.%, particularly preferably 0.5 to 5 wt.% water, in each case based on the weight of the oxidative hair lightening agent,
e) wherein no silicone compounds are present.

2. The oxidative hair lightening agent according to claim 1,
**characterized in that** xanthan gum is present in an amount of 0.3 - 5 wt.%, preferably 1 - 4 wt.%, particularly preferably 1.5 - 3 wt.%, extremely preferably 2 - 2.5 wt.%, in each case based on the weight of the oxidative hair lightening agent.

3. The oxidative hair lightening agent according to claim 1 or 2, **characterized in that** microcrystalline cellulose is present in an amount of 0.01 - 5 wt.%, preferably 0.02 - 2 wt.%, particularly preferably 0.04 - 0.5 wt.%, extremely preferably 0.05 - 0.1 wt.%, in each case based on the weight of the oxidative hair lightening agent.

4. The oxidative hair lightening agent according to one of claims 1 - 3, **characterized in that** guar gum is present in an amount of 0.01 - 5 wt.%, preferably 0.02 - 2 wt.%, particularly preferably 0.04 - 0.5 wt.%, extremely preferably 0.05 - 0.1 wt.%, in each case based on the weight of the oxidative hair lightening agent.

5. The oxidative hair lightening agent according to one of claims 1 - 4, **characterized in that** the at least one oxidizing agent a) is selected from sodium peroxydisulfate, potassium peroxydisulfate, ammonium peroxydisulfate, sodium peroxymonosulfate, potassium peroxymonosulfate, ammonium peroxymonosulfate and mixtures of these compounds, preferably potassium peroxydisulfate and ammonium peroxydisulfate mixtures, potassium peroxydisulfate, ammonium peroxydisulfate and sodium peroxydisulfate mixtures, and potassium peroxydisulfate and sodium peroxydisulfate mixtures, particularly preferably from potassium peroxydisulfate and ammonium peroxydisulfate mixtures, extremely preferably from potassium peroxydisulfate and ammonium peroxydisulfate mixtures with an excess of potassium peroxydisulfate, which are free of sodium peroxydisulfate.

6. The oxidative hair lightening agent according to one of claims 1 - 5, **characterized in that** the at least one oxidizing agent a) is present in a total amount of 5 - 85 wt.%, preferably 10 - 70 wt.%, particularly preferably 17 - 55 wt.%, and extremely preferably 30 - 45 wt.%, in each case based on the weight of the oxidative hair lightening agent.

7. The oxidative hair lightening agent according to one of claims 1 - 6, **characterized in that** at least one complexing agent is also present, selected from complexing agents of the general formula (I), 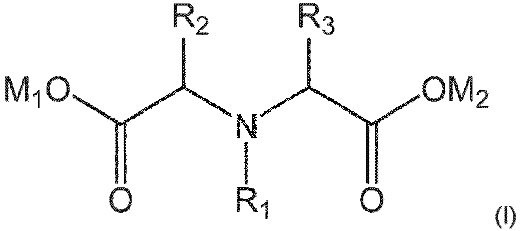 where
R1 represents a hydrogen atom, a C₁-C₆-alkyl group, a hydroxy-C₂-C₆-alkyl group, a carboxy-C₁-C₆-alkyl group, or a physiologically acceptable salt thereof,
R2, R3 represent, independently of one another, a hydrogen atom, a C₁-C₆-alkyl group, a hydroxy-C₂-C₆-alkyl group, a carboxy-C₁-C₆-alkyl group or a physiologically acceptable salt thereof, where R2 and R3 do not simultaneously represent a hydrogen atom and do not simultaneously represent a carboxy-C₁-C₆-alkyl group, M1, M2 represent, independently of one another, a hydrogen atom or an alkali metal ion, preferably sodium, potassium, or an ammonium ion (NH₄⁺).

8. The oxidative hair lightening agent according to claim 7,
**characterized in that** the at least one complexing agent of the general formula (I) is selected from methylglycine diacetic acid, the sodium salts, potassium salts and ammonium salts of methylglycine diacetic acid, and mixtures of the aforementioned substances, the disodium salt and the trisodium salt of methylglycine diacetic acid being preferred and the trisodium salt of methylglycine diacetic acid being particularly preferred.

9. The oxidative hair lightening agent according to one of claims 7 or 8, **characterized in that** the at least one complexing agent of the general formula (I) is present in a total amount of 0.1 - 5.0 wt.%, preferably 0.2 - 2.0 wt.%, particularly preferably 0.4 - 1.6 wt.%, extremely preferably 0.5 - 1.0 wt.%, in each case based on the weight of the oxidative hair lightening agent.

10. The oxidative hair lightening agent according to one of claims 7 - 9, **characterized in that** the trisodium salt of methylglycine diacetic acid is present in an amount of 0.1 to 5.0 wt.%, preferably 0.2 to 2.0 wt.%, particularly preferably 0.4 to 1.6 wt.%, extremely preferably 0.5 to 1.0 wt.%, in each case based on the weight of the oxidative hair lightening agent.

11. The oxidative hair lightening agent according to one of claims 1 - 10, **characterized in that** ammonium chloride is also present, preferably in an amount of 0.1 to 10.0 wt.%, preferably 1.0 to 8.0 wt.%, particularly preferably 2.0 to 6.0 wt.%, extremely preferably 3.1 - 5.0 wt.%, in each case based on the weight of the oxidative hair lightening agent.

12. The oxidative hair lightening agent according to one of claims 1 - 11, **characterized in that** 0.05 - 0.5 wt.%, preferably 0.2 - 0.4 wt.% sodium hexametaphosphate is present, in each case based on the weight of the oxidative hair lightening agent.

13. The oxidative hair lightening agent according to one of claims 1 - 12, **characterized in that** at least one inorganic alkalizing agent, solid at 20 °C and 1013 mbar (101.3 kPa), is present in a total amount of 4 - 70 wt.%, preferably 10 - 60 wt.%, particularly preferably 15 - 50 wt.%, further particularly preferably 20 - 55 wt.%, and extremely preferably 30 - 45 wt.%, in each case based on the weight of the oxidative hair lightening agent, in each case based on the weight of the oxidative hair lightening agent.

14. A method for lightening or coloring keratin fibers, in particular human hair, lighter, **characterized in that** an oxidative hair lightening agent (B) according to one of claims 1 - 13 is mixed with an oxidizing composition (Ox), which, in each case based on its weight, contains 50 - 96 wt.%, preferably 70 - 93 wt.%, particularly preferably 80 - 90 wt.% water and 0.5 - 20 wt.% hydrogen peroxide and also contains at least one pH setting agent in an amount such that the oxidizing composition has a pH in the range of 1.5 to 5.5, measured at 20 °C, said lightening agent is applied immediately afterward to the keratin-containing fibers, is left on the fibers for 5 to 60 minutes, and is subsequently rinsed from the fibers with water and optionally washed with a surfactant-containing cleaning agent, the oxidative hair lightening agent (B) and the oxidizing composition (Ox) being mixed with one another, preferably in a weight-related ratio (B):(Ox) of 0.2 - 2, particularly preferably 0.3 - 1.5, more preferably 0.5 - 1.

15. A multi-component packaging unit (kit-of-parts) for lightening keratin fibers which contains at least two components packaged separately from one another and is **characterized in that**
i) the first component (B) is an oxidative hair treatment agent according to one of claims 1 - 13,
ii) the second component (Ox) is an oxidizing composition which contains, in each case based on its weight, 50 - 96 wt.%, preferably 70 - 93 wt.%, particularly preferably 80 - 90 wt.% water and 0.5 - 20 wt.% hydrogen peroxide and also contains at least one pH setting agent in an amount such that the oxidizing composition has a pH in the range of 1.5 to 5.5, measured at 20 °C,
components (B) and (Ox) preferably being present in a weight-based ratio (B):(Ox) to one another of 0.2 - 2, particularly preferably 0.3 - 1.5, more preferably 0.5 - 1, extremely preferably 0.5 - 0.6.

## Revendications

1. Agent oxydant d'éclaircissement capillaire permettant d'éclaircir ou de teindre par éclaircissement les cheveux, lequel se présente sous forme de poudre non compressée ou de pâte fluide, contenant
a) au moins un agent oxydant choisi parmi les sels inorganiques d'un acide peroxysulfurique, ainsi que des mélanges de ces sels,
b) de la gomme xanthane,
c) un polysaccharide choisi parmi la cellulose microcristalline et les mélanges de cellulose microcristalline et de gomme de guar,
d) et 0 à 10 % en poids, de préférence 0,1 à 8 % en poids, de manière particulièrement préférée 0,5 à 5 % en poids, d'eau, respectivement par rapport au poids de l'agent oxydant d'éclaircissement capillaire,
e) dans lequel aucun composé de silicone n'est contenu.

2. Agent oxydant d'éclaircissement capillaire selon la revendication 1, **caractérisé en ce que** la gomme xanthane est contenue en une quantité allant de 0,3 à 5 % en poids, de préférence de 1 à 4 % en poids, de manière particulièrement préférée de 1,5 à 3 % en poids, de manière extrêmement préférée de 2 à 2,5 % en poids, respectivement par rapport au poids de l'agent oxydant d'éclaircissement capillaire.

3. Agent oxydant d'éclaircissement capillaire selon la revendication 1 ou 2, **caractérisé en ce que** la cellulose microcristalline est contenue en une quantité allant de 0,01 à 5 % en poids, de préférence de 0,02 à 2 % en poids, de manière particulièrement préférée de 0,04 à 0,5 % en poids, de manière extrêmement préférée de 0,05 à 0,1 % en poids, respectivement par rapport au poids de l'agent oxydant d'éclaircissement capillaire.

4. Agent oxydant d'éclaircissement capillaire selon l'une des revendications 1 à 3, **caractérisé en ce que** la gomme de guar est contenue en une quantité allant de 0,01 à 5 % en poids, de préférence de 0,02 à 2 % en poids, de manière particulièrement préférée de 0,04 à 0,5 % en poids, de manière extrêmement préférée de 0,05 à 0,1 % en poids, respectivement par rapport au poids de l'agent oxydant d'éclaircissement capillaire.

5. Agent oxydant d'éclaircissement capillaire selon l'une des revendications 1 à 4, **caractérisé en ce que** l'au moins un agent oxydant a) est choisi parmi peroxodisulfate de sodium, peroxodisulfate de potassium, peroxodisulfate d'ammonium, peroxomonosulfate de sodium, peroxomonosulfate de potassium, peroxomonosulfate d'ammonium ainsi que mélanges de ces composés, de préférence mélanges de peroxodisulfate de potassium et de peroxodisulfate d'ammonium, mélanges de peroxodisulfate de potassium, de peroxodisulfate d'ammonium et de peroxodisulfate de sodium, et mélanges de peroxodisulfate de potassium et de peroxodisulfate de sodium, de manière particulièrement préférée parmi mélanges de peroxodisulfate de potassium et de peroxodisulfate d'ammonium, de manière extrêmement préférée parmi mélanges de peroxodisulfate de potassium et de peroxodisulfate d'ammonium comportant un excès de peroxodisulfate de potassium, qui sont exempts de peroxodisulfate de sodium.

6. Agent oxydant d'éclaircissement capillaire selon l'une des revendications 1 à 5, **caractérisé en ce que** l'au moins un agent oxydant a) est contenu en une quantité totale allant de 5 à 85 % en poids, de préférence de 10 à 70 % en poids, de manière particulièrement préférée de 17 à 55 % en poids, de manière extrêmement préférée de 30 à 45 % en poids, respectivement par rapport au poids de l'agent oxydant d'éclaircissement capillaire.

7. Agent oxydant d'éclaircissement capillaire selon l'une des revendications 1 à 6, **caractérisé en ce qu'**au moins un agent complexant est en outre contenu, lequel est choisi parmi les agents complexants de formule générale (I), 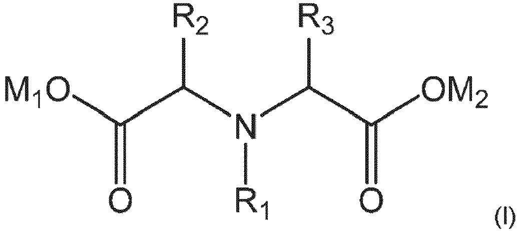 où
R1 représente un atome d'hydrogène, un groupe alkyle en C₁-C₆, un groupe hydroxyalkyle en C₂-C₆, un groupe carboxyalkyle en C₁-C₆ ou un sel physiologiquement acceptable de ceux-ci,
R2, R3 représentent, indépendamment l'un de l'autre, un atome d'hydrogène, un groupe alkyle en C₁-C₆, un groupe hydroxyalkyle en C₂-C₆, un groupe carboxyalkyle en C₁-C₆ ou un sel physiologiquement acceptable de ceux-ci, où R2 et R3 ne représentent pas simultanément un atome d'hydrogène ni simultanément un groupe carboxyalkyle en C₁-C₆,
M1, M2 représentent, indépendamment l'un de l'autre, un atome d'hydrogène ou un ion de métal alcalin, de préférence sodium, potassium ou ion ammonium (NH₄⁺).

8. Agent oxydant d'éclaircissement capillaire selon la revendication 7, **caractérisé en ce que** l'au moins un agent complexant de formule générale (I) est choisi parmi acide méthylglycinediacétique, sels de sodium, sels de potassium et sels d'ammonium de l'acide méthylglycinediacétique, ainsi que mélanges des substances susmentionnées, dans lequel le sel disodique et le sel trisodique de l'acide méthylglycinediacétique sont préférés, et le sel trisodique de l'acide méthylglycinediacétique est particulièrement préféré.

9. Agent oxydant d'éclaircissement capillaire selon l'une des revendications 7 ou 8, **caractérisé en ce que** l'au moins un agent complexant de formule générale (I) est contenu en une quantité totale allant de 0,1 à 5,0 % en poids, de préférence de 0,2 à 2,0 % en poids, de manière particulièrement préférée de 0,4 à 1,6 % en poids, de manière extrêmement préférée de 0,5 à 1,0 % en poids, respectivement par rapport au poids de l'agent oxydant d'éclaircissement capillaire.

10. Agent oxydant d'éclaircissement capillaire selon l'une des revendications 7 à 9, **caractérisé en ce que** le sel trisodique de l'acide méthylglycinediacétique est contenu en une quantité allant de 0,1 à 5,0 % en poids, de préférence de 0,2 à 2,0 % en poids, de manière particulièrement préférée de 0,4 à 1,6 % en poids, de manière extrêmement préférée de 0,5 à 1,0 % en poids, respectivement par rapport au poids de l'agent oxydant d'éclaircissement capillaire.

11. Agent oxydant d'éclaircissement capillaire selon l'une des revendications 1 à 10, **caractérisé en ce que** du chlorure d'ammonium est en outre contenu, de préférence en une quantité allant de 0,1 à 10,0 % en poids, de préférence de 1,0 à 8,0 % en poids, de manière particulièrement préférée de 2,0 à 6,0 % en poids, de manière extrêmement préférée de 3,1 à 5,0 % en poids, respectivement par rapport au poids de l'agent oxydant d'éclaircissement capillaire.

12. Agent oxydant d'éclaircissement capillaire selon l'une des revendications 1 à 11, **caractérisé en ce qu'**il contient 0,05 à 0,5 % en poids, de préférence 0,2 à 0,4 % en poids, d'hexamétaphosphate de sodium, respectivement par rapport au poids de l'agent oxydant d'éclaircissement capillaire.

13. Agent oxydant d'éclaircissement capillaire selon l'une des revendications 1 à 12, **caractérisé en ce qu'**au moins un agent alcalinisant inorganique et solide à 20 °C et 1013 mbar (101,3 kPa), est contenu en une quantité totale allant de 4 à 70 % en poids, de préférence de 10 à 60 % en poids, de manière particulièrement préférée de 15 à 50 % en poids, de manière plus particulièrement préférée de 20 à 55 % en poids, de manière extrêmement préférée de 30 à 45 % en poids, respectivement par rapport au poids de l'agent oxydant d'éclaircissement capillaire, respectivement par rapport au poids de l'agent oxydant d'éclaircissement capillaire.

14. Procédé permettant d'éclaircir ou de teindre par éclaircissement des fibres kératiniques, en particulier des cheveux humains, **caractérisé en ce qu'**un agent oxydant d'éclaircissement capillaire (B) selon l'une des revendications 1 à 13 est mélangé à une composition oxydante (Ox) qui contient, respectivement par rapport à son poids, 50 à 96 % en poids, de préférence 70 à 93 % en poids, de manière particulièrement préférée 80 à 90 % en poids, d'eau et 0,5 à 20 % en poids de peroxyde d'hydrogène et en outre au moins un agent de régulation du pH en une quantité telle que la composition oxydante présente un pH compris dans la plage allant de 1,5 à 5,5, mesuré à 20 °C, puis est appliqué immédiatement après sur les fibres contenant de la kératine, est laissé sur les fibres pendant 5 à 60 minutes, puis les fibres sont rincées à l'eau et éventuellement lavées avec un agent nettoyant contenant un tensioactif, dans lequel l'agent oxydant d'éclaircissement capillaire (B) et la composition oxydante (Ox) sont mélangés l'un à l'autre de préférence dans un rapport pondéral (B) : (Ox) de 0,2 à 2, de manière particulièrement préférée de 0,3 à 1,5, de manière plus particulièrement préférée de 0,5 à 1.

15. Unité de conditionnement à plusieurs composants (kit de pièces) permettant d'éclaircir des fibres kératiniques, laquelle contient au moins deux composants conditionnés séparément l'un de l'autre et est
**caractérisée en ce que**
i) le premier composant (B) est un agent oxydant d'éclaircissement capillaire selon l'une des revendications 1 à 13,
ii) le second composant (Ox) est une composition oxydante qui contient, respectivement par rapport à son poids, 50 à 96 % en poids, de préférence 70 à 93 % en poids, de manière particulièrement préférée 80 à 90 % en poids, d'eau, et 0,5 à 20 % en poids de peroxyde d'hydrogène et en outre au moins un agent de régulation du pH en une quantité telle que la composition oxydante présente un pH compris dans la plage allant de 1,5 à 5,5, mesuré à 20 °C,
dans laquelle les composants (B) et (Ox) sont de préférence présents l'un par rapport à l'autre dans un rapport pondéral (B) : (Ox) de 0,2 à 2, de manière particulièrement préférée de 0,3 à 1,5, de manière plus particulièrement préférée de 0,5 à 1, de manière extrêmement préférée de 0,5 à 0,6.
